# EUROPEAN PATENT APPLICATION

(11) **EP 4 282 413 A1**
(43) Date of publication of application: **29.11.2023**
(21) Application number: 22742206.0
(22) Date of filing: 20.01.2022
(51) Int. Cl.: A61K 31/44, A61K 39/395, A61P 35/00, A61P 35/04

(54) **COMPOSITION AND METHOD FOR TREATING TUMORS**

(30) Priority: 21.01.2021 CN 202110078975
(71) Applicant: NATURAL MEDICINE INSTITUTE OF ZHEJIANG YANGSHENGTANG CO., LTD., Xihu District Hangzhou Zhejiang 310024 (CN)
(72) Inventor: XU, Nuo, Hangzhou, Zhejiang 310024 (CN); LIN, Jian, Beijing 100871 (CN)
(74) Representative: Weickmann & Weickmann PartmbB
(86) International application number: PCT/CN2022/072887
(87) International publication number: WO 2022/156727

(57) **Abstract**

The present invention relates to a composition and a method for treating tumors. The method comprises administering an effective amount of one or more immune checkpoint inhibitors, and an effective amount of a PDE4-selective inhibitor. The composition and the method have a significant anti-tumor effect.

## Description

### Field of the Invention

The present application relates to the field of biomedicine, and specifically relates to a composition and a method for treating tumors.

### Background of the Invention

Malignant tumor is currently a disease severely endangering human health. The results of global cancer statistics in 2018 show that there were 18.19 million new cases of cancer and 9.6 million cancer deaths worldwide. Taking colorectal cancer as an example, the colorectal cancer accounts for 10.2% of the total new cases of cancer and 9.2% of the cancer deaths, that is, there are about 1.85 million new cases of colorectal cancer and 880,000 deaths every year. There are about 370,000 new cases of colon cancer in China every year. Clinical therapy for colorectal cancer is mostly based on oxaliplatin, and clinical chemotherapy regimens comprise oxaliplatin in combination with Xeloda and oxaliplatin in combination with fluorouracil. However, these chemotherapy regimens have serious side effects, such as, damage to the digestive system and blood system, and often develop drug resistance. In addition, more than 30% of patients with colon cancer are not clinically sensitive to platinum-based chemotherapy drugs. All these show that chemotherapy drugs exhibit different degrees of toxic side effects or drug resistance defects while having anti-tumor effects, especially some tumor patients are not sensitive to chemotherapy drugs. Therefore, it is an urgent problem to find more effective antitumor drugs and methods.

### Summary Of The Invention

The present application provides a pharmaceutical combination and a method for treating tumors, mainly comprising administering an effective amount of a PDE4 (type 4 phosphodiesterase) selective inhibitor. In certain instances, the pharmaceutical combination or method of the present application further comprises administering an effective amount of an immune checkpoint inhibitor. The pharmaceutical combination or method of the present application has one or more of 1) avoidance of side effects produced by chemotherapy; 2) effective inhibition of tumor (cell) growth, such as solid tumors (e.g., colorectal cancer), lymphomas, and/or hematological tumors; 3) inhibition of tumor cell metastasis (e.g., inhibition of liver metastasis of the tumor); 4) no significant effect on other physiological signs (e.g., body weight) of a subject, and safety.

The present application provides a pharmaceutical combination comprising:
a) an effective amount of an immune checkpoint inhibitor; and
b) an effective amount of a PDE4-selective inhibitor.

In certain embodiments, wherein the PDE4-selective inhibitor comprises Apremilast, Crisaborole, Drotaverine, CC-1088, BPN14770, GSK356278, HT-0712, TAK-648, Zembrin, ASP9831, Etazolate, Piclamilast, Rolipram, Dipyridamole, Cilomilast, GSK256066, AWD 12-281, Quinolyl oxazole, DC-TA-46, Filaminast, and/or Glaucine.

In certain embodiments, wherein the PDE4-selective inhibitor comprises a compound of Formula I, a pharmaceutically acceptable salt thereof, or an N-oxide of pyridine or a salt thereof: 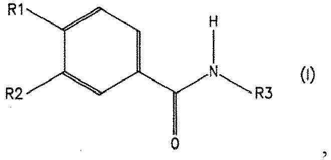 wherein one of R1 and R2 is hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 is C₁-C₄-alkoxy fully or partially substituted with fluorine, and
R3 is phenyl, pyridinyl, phenyl substituted with R31, R32 and R33, or pyridinyl substituted with R34, R35, R36 and R37, wherein R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein,
R1 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein:
R1 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine,
R2 is methoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein:
R1 is C₃-C₇-cycloalkoxy, C₃-C₇-cycloalkylmethoxy or benzyloxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein:
R1 is C₁-C₄-alkoxy fully or partially substituted with fluorine,
R2 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein:
R1 is C₁-C₄-alkoxy fully or partially substituted with fluorine,
R2 is C₃-C₇-cycloalkylmethoxy or benzyloxy, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

In certain embodiments, wherein:
R1 is C₁-C₄-alkoxy, C₃-C₅-cycloalkoxy, Cs-Cs-cycloalkylmethoxy, benzyloxy or C₁-C₄-alkoxy fully or partially substituted with fluorine,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₁-C₄-alkoxy, C₃-C₅-cycloalkoxy, Cs-Cs-cycloalkylmethoxy, benzyloxy or C₁-C₄-alkoxy fully or partially substituted with fluorine,
R2 is methoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₃-C₅-cycloalkoxy, Cs-Cs-cycloalkylmethoxy or benzyloxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₁-C₄-alkoxy fully or partially substituted with fluorine,
R2 is C₁-C₄-alkoxy, C₃-C₅-cycloalkoxy, C₃-C₅-cycloalkylmethoxy, benzyloxy or C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₁-C₄-alkoxy fully or partially substituted with fluorine,
R2 is Cs-Cs-cycloalkylmethoxy or benzyloxy, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₁-C₄-alkoxy, C₃-C₅-cycloalkoxy, Cs-Cs-cycloalkylmethoxy, benzyloxy or C₁-C₄-alkoxy fully or partially substituted with fluorine,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₁-C₄-alkoxy, C₃-C₅-cycloalkoxy, Cs-Cs-cycloalkylmethoxy, benzyloxy or C₁-C₄-alkoxy fully or partially substituted with fluorine,
R2 is methoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₃-C₅-cycloalkoxy, Cs-Cs-cycloalkylmethoxy or benzyloxy,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₁-C₄-alkoxy fully or partially substituted with fluorine,
R2 is C₁-C₄-alkoxy, C₃-C₅-cycloalkoxy, Cs-Cs-cycloalkylmethoxy, benzyloxy or C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₁-C₄-alkoxy fully or partially substituted with fluorine,
R2 is Cs-Cs-cycloalkylmethoxy or benzyloxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is difluoromethoxy,
R2 is methoxy, ethoxy, isopropoxy, isobutoxy, cyclopentoxy, cyclopropylmethoxy, cyclobutylmethoxy, difluoromethoxy or 2,2,2-trifluoroethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is difluoromethoxy,
R2 is methoxy, cyclopropylmethoxy, cyclobutylmethoxy or difluoromethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is methoxy, n-propoxy, n-butoxy, cyclopropylmethoxy or 2,2,2-trifluoroethoxy,
R2 is difluoromethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is difluoromethoxy,
R2 is cyclopropylmethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₃-C₇-cycloalkoxy or C₃-C₇-cycloalkylmethoxy, and R2 is C₁-C₄-alkoxy fully or partially substituted with fluorine; or R1 is C₁-C₄-alkoxy fully or partially substituted with fluorine, and R2 is C₃-C₇-cycloalkylmethoxy; and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C4-alkyl, or C₁-C₄-alkoxy, R34 is hydroxy, halogen, carboxyl, C1-C4-alkyl, C₁-C₄-alkoxy, or C₁-C₄-alkoxycarbonyl, R35 is hydrogen, halogen, amino, or C₁-C₄-alkyl, R36 is hydrogen or halogen, and R37 is hydrogen or halogen.

In certain embodiments, wherein:
R1 is C₃-C₇-cycloalkoxy or C₃-C₇-cycloalkylmethoxy,
R2 is C₁-C₄-alkoxy fully or partially substituted with fluorine, and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C4-alkyl, or C₁-C₄-alkoxy, R34 is hydroxy, halogen, carboxyl, C1-C4-alkyl, C₁-C₄-alkoxy, or C₁-C₄-alkoxycarbonyl, R35 is hydrogen, halogen, amino, or C₁-C₄-alkyl, R36 is hydrogen or halogen, and R37 is hydrogen or halogen.

In certain embodiments, wherein:
R1 is C₁-C₄-alkoxy fully or partially substituted with fluorine,
R2 is C₃-C₇-cycloalkylmethoxy, and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C4-alkyl, or C₁-C₄-alkoxy, R34 is hydroxy, halogen, carboxyl, C1-C4-alkyl, C₁-C₄-alkoxy, or C₁-C₄-alkoxycarbonyl, R35 is hydrogen, halogen, or C₁-C₄-alkyl, R36 is hydrogen or halogen, and R37 is hydrogen or halogen.

In certain embodiments, wherein:
R1 is C₃-C₅-cycloalkoxy or C₃-C₅-cycloalkylmethoxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₁-C₄-alkoxy fully or partially substituted with fluorine,
R2 is Cs-Cs-cycloalkylmethoxy, and
R3 is phenyl, pyridinyl, phenyl substituted with R31, R32 and R33 or pyridinyl substituted with R34, R35, R36 and R37, wherein R31 is halogen, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

In certain embodiments, wherein:
R1 is C₃-C₅-cycloalkoxy, Cs-Cs-cycloalkylmethoxy or benzyloxy,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is C₁-C₄-alkoxy fully or partially substituted with fluorine,
R2 is Cs-Cs-cycloalkylmethoxy, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein:
R1 is difluoromethoxy,
R2 is cyclopropylmethoxy, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In certain embodiments, wherein R1 is difluoromethoxy, R2 is cyclopropylmethoxy, and R3 is 3,5-dichloropyridin-4-yl.

In certain embodiments, wherein the PDE4-selective inhibitor comprises Roflumilast or a derivative thereof.

In certain embodiments, wherein the derivative of Roflumilast comprises Benzanilide, 3-hydroxy-N-(3,5-dichloropyridin-4-yl)-4-methoxybenzamide and/or 4-Methylbenzanilide.

In certain embodiments, wherein the immune checkpoint inhibitor comprises an antagonist of one or more proteins of CTLA-4, PD-1, PD-L1, PD-L2, LAG-3, TIM-3, galectin-9, CEACAM-1, BTLA, CD69, galectin-1, TIGIT, CD113, GPR56, VISTA, 2B4, CD48, GARP, PD1H, LAIR1, TIM-1, and TIM-4.

In certain embodiments, wherein the immune checkpoint inhibitor comprises an antagonist of PD-1 and/or PD-L1.

In certain embodiments, wherein the antagonist of PD-1 and/or PD-L1 can inhibit an interaction between PD-1 and PD-L1.

In certain embodiments, wherein the antagonist of PD-1 and/or PD-L1 comprises an antibody specifically binding to PD-1 and/or PD-L1 or an antigen binding fragment thereof.

In certain embodiments, wherein the antibody specifically binding to PD-1 and/or PD-L1 or antigen binding fragment thereof comprises a HCDR3, and the HCDR3 comprises an amino acid sequence set forth in any one of SEQ ID NOs: 3, 13, 23, and 33.

In certain embodiments, wherein the antibody specifically binding to PD-1 and/or PD-L1 or antigen binding fragment thereof comprises a HCDR2, and the HCDR2 comprises an amino acid sequence set forth in any one of SEQ ID NOs: 2, 12, 22, and 32.

In certain embodiments, wherein the antibody specifically binding to PD-1 and/or PD-L1 or antigen binding fragment thereof comprises a HCDR1 , and the HCDR1 comprises an amino acid sequence set forth in any one of SEQ ID NOs: 1, 11, 21, and 31.

In certain embodiments, wherein the antibody specifically binding to PD-1 and/or PD-L1 or antigen binding fragment thereof comprises a VH , and the VH comprises an amino acid sequence set forth in any one of SEQ ID NOs: 7, 17, 27, and 37.

In certain embodiments, wherein the antibody specifically binding to PD-1 and/or PD-L1 or antigen binding fragment thereof comprises an LCDR3, and the LCDR3 comprises an amino acid sequence set forth in any one of SEQ ID NOs: 6, 16, 26, and 36.

In certain embodiments, wherein the antibody specifically binding to PD-1 and/or PD-L1 or antigen binding fragment thereof comprises an LCDR2, and the LCDR2 comprises an amino acid sequence set forth in any one of SEQ ID NOs: 5, 15, 25, and 35.

In certain embodiments, wherein the antibody specifically binding to PD-1 and/or PD-L1 or antigen binding fragment thereof comprises an LCDR1, and the LCDR1 comprises an amino acid sequence set forth in any one of SEQ ID NOs: 4, 14, 24, and 34.

In certain embodiments, wherein the antibody specifically binding to PD-1 and/or PD-L1 or antigen binding fragment thereof comprises a VL, and the VL comprises an amino acid sequence set forth in any one of SEQ ID NOs: 8, 18, 28, and 38.

In certain embodiments, wherein the antibody specifically binds to PD-1 and/or PD-L1 is selected from the group consisting of:
a) an antibody comprising the following heavy chain and light chain, the heavy chain comprises an amino acid sequence set forth in any one of SEQ ID NOs: 9, 19, 29, and 39, and the light chain comprises an amino acid sequence set forth in any one of SEQ ID NOs: 10, 20, 30, and 40;
b) an antibody comprising the following heavy chain and light chain, the heavy chain comprises a VH having an amino acid sequence set forth in any one of SEQ ID NOs: 7, 17, 27 and 37, and the light chain comprises a VL having an amino acid sequence set forth in any one of SEQ ID NOs: 8, 18, 28 and 38; and
c) an antibody comprising the following heavy chain and light chain, the heavy chain comprises a HCDR1 having an amino acid sequence set forth in any one of SEQ ID NOs: 1, 11, 21, and 31, a HCDR2 having an amino acid sequence set forth in any one of SEQ ID NOs: 2, 12, 22, and 32, and a HCDR3 having an amino acid sequence set forth in any one of SEQ ID NOs: 3, 13, 23, and 33, and the light chain comprises an LCDR1 having an amino acid sequence set forth in any one of SEQ ID NOs: 4, 14, 24, and 34, an LCDR2 having an amino acid sequence set forth in any one of SEQ ID NOs: 5, 15, 25, and 35, and an LCDR3 having an amino acid sequence set forth in any one of SEQ ID NOs: 6, 16, 26, and 36.

In certain embodiments, wherein the antibody is selected from: a chimeric antibody, a humanized antibody, and a fully human antibody.

In certain embodiments, wherein the antigen binding fragment is selected from: Fab, Fv, scFv, Fab ', and (Fab')₂.

In certain embodiments, wherein the antibody specifically binding to PD-1 and/or PD-L1 comprises:
HCDR1-3 and LCDR1-3 of nivolumab,
HCDR1-3 and LCDR1-3 of pembrolizumab,
HCDR1-3 and LCDR1-3 of BMS-936559;
HCDR1-3 and LCDR1-3 of MPDL3280A;
HCDR1-3 and LCDR1-3 of MEDI4736; or
HCDR1-3 and LCDR1-3 of MSB0010718C.

In certain embodiments, wherein the antibody specifically binding to PD-1 and/or PD-L1 comprises:
a VH and a VL of nivolumab,
a VH and a VL of pembrolizumab,
a VH and a VL of BMS-936559;
a VH and a VL of MPDL3280A;
a VH and a VL of MEDI4736; or
a VH and a VL of MSB0010718C.

In certain embodiments, wherein the antibody specifically binds to PD-1 and/or PD-L1 is selected from the group consisting of nivolumab, pembrolizumab, BMS-936559, MPDL3280A, MEDI4736, AMP-224, and MSB0010718C.

In certain embodiments, wherein a ratio of an effective amount of the PDE4-selective inhibitor to an effective amount of the immune checkpoint inhibitor is from about 9:1 to about 1:4 (mass ratio).

In certain embodiments, wherein the PDE4-selective inhibitor and the immune checkpoint inhibitor are present in different containers, respectively.

In certain embodiments, wherein the pharmaceutical combination comprises a first formulation comprising the immune checkpoint inhibitor and a first pharmaceutically acceptable carrier, and a second formulation comprising the PDE4-selective inhibitor and a second pharmaceutically acceptable carrier.

In certain embodiments, wherein the PDE4-selective inhibitor is contained in the second formulation in an amount of about 20% (w/w) to about 90% (w/w).

In certain embodiments, wherein the immune checkpoint inhibitor is contained in the first formulation in an amount of about 10% (w/w) to about 80% (w/w).

In certain embodiments, the pharmaceutical combination comprises a pharmaceutical composition comprising the PDE4-selective inhibitor and the immune checkpoint inhibitor.

In certain embodiments, wherein the PDE4-selective inhibitor is contained in the pharmaceutical composition in an amount of about 20% (w/w) to about 90% (w/w).

In certain embodiments, wherein the immune checkpoint inhibitor is contained in the pharmaceutical composition in an amount of about 10% (w/w) to about 80% (w/w).

In another aspect, the present application also provides a kit comprising the pharmaceutical combination of the present application.

The pharmaceutical combination or kit according to the present application for preventing and/or treating a tumor.

The pharmaceutical combination or kit according to the present application, wherein the tumor comprises a solid tumor, a hematological tumor, and/or a lymphoma.

The pharmaceutical combination or kit according to the present application, wherein the tumor comprises colon cancer, breast cancer, and/or melanoma.

The pharmaceutical combination or kit according to the present application, wherein the tumor comprises a metastatic tumor.

The pharmaceutical combination or kit according to the present application, wherein the tumor comprises a tumor with liver metastasis.

The pharmaceutical combination or kit according to the present application, wherein the tumor comprises colon cancer with liver metastasis.

In another aspect, the present application also provides a PDE4-selective inhibitor for preventing and/or treating a tumor.

The PDE4-selective inhibitor according to the present application for preventing and/or treating a tumor in combination with the immune checkpoint inhibitor according to the present application.

The PDE4-selective inhibitor according to the present application, wherein the PDE4-selective inhibitor is the PDE4-selective inhibitor according to the present application.

The PDE4-selective inhibitor according to the present application, wherein the tumor comprises a solid tumor, a hematological tumor, and/or a lymphoma.

The PDE4-selective inhibitor according to the present application, wherein the tumor comprises colon cancer, breast cancer, and/or melanoma.

The PDE4-selective inhibitor according to the present application, wherein the tumor comprises a metastatic tumor.

The PDE4-selective inhibitor according to the present application, wherein the tumor comprises a tumor with liver metastasis.

The PDE4-selective inhibitor according to the present application, wherein the tumor comprises colon cancer with liver metastasis.

In another aspect, the present application also provides a method for preventing and/or treating a tumor, comprising administering to a subject in need thereof an effective amount of the PDE4-selective inhibitor of the present application.

The method according to the present application, further comprising administering to the subject an effective amount of the immune checkpoint inhibitor of the present application.

The method according to the present application, wherein the PDE4-selective inhibitor is administered simultaneously or sequentially with the immune checkpoint inhibitor.

The method according to the present application, wherein the tumor comprises a solid tumor, a hematological tumor, and/or a lymphoma.

The method according to the present application, wherein the tumor comprises colon cancer, breast cancer, and/or melanoma.

The method according to the present application, wherein the tumor comprises a metastatic tumor.

The method according to the present application, wherein the tumor comprises a tumor with liver metastasis.

The method according to the present application, wherein the tumor comprises colon cancer with liver metastasis.

In another aspect, the present application also provides use of a PDE4-selective inhibitor in preparation of a medicament for preventing and/or treating a tumor.

In another aspect, the present application also provides use of a PDE4-selective inhibitor in combination with an immune checkpoint inhibitor in preparation of a medicament for preventing and/or treating a tumor.

The use according to the present application, wherein the PDE4-selective inhibitor is the PDE4-selective inhibitor according to the present application.

The use according to the present application, wherein the immune checkpoint inhibitor is the immune checkpoint inhibitor of the present application.

The use according to the present application, wherein the tumor comprises a solid tumor, a hematological tumor, and/or a lymphoma.

The use according to the present application, wherein the tumor comprises colon cancer, breast cancer, and/or melanoma.

The use according to the present application, wherein the tumor comprises a metastatic tumor.

The use according to the present application, wherein the tumor comprises a tumor with liver metastasis.

The use according to the present application, wherein the tumor comprises colon cancer with liver metastasis.

Other aspects and advantages of the present application can be readily perceived by those skilled in the art from the detailed description below. Only exemplary embodiments of the present application are shown and described in the following detailed description. As will be appreciated by those skilled in the art, the contents of the present application enable those skilled in the art to make changes to the disclosed specific embodiments without departing from the spirit and scope of the invention to which the present application relates. Accordingly, accompanying drawings of the present application and the description in the specification are merely exemplary and not limiting.

### Brief Description Of The Drawings

Specific features of the invention to which the present application relates are shown in the appended claims. The features and advantages of the invention to which the present application relates can be better understood with reference to the exemplary embodiments described in detail hereinafter and the accompanying drawings. The accompanying drawings are briefly illustrated as follows:
FIGs. 1A and 1B show the effect of the PDE4-selective inhibitor described in the present application in inhibiting tumor cell metastasis.
FIGs. 2A, 2B, and 2C show the *in vivo* effect of the PDE4-selective inhibitor in combination with the immune checkpoint inhibitor described in the present application in the treatment of colon cancer.
FIG. 3 shows the result of metastasis inhibition by Roflumilast. FIG. 4 shows the result of metastasis inhibition by BPN14770.
FIG. 5 shows the result of metastasis inhibition by GSK256066. FIG. 6 shows the result of metastasis inhibition by Rolipram. FIG. 7 shows the result of metastasis inhibition by Dipyridamole. FIG. 8 shows the inhibitory effect of Roflumilast on tumor metastasis.
FIG. 9 shows the inhibitory effect of a PDE4 inhibitor in combination with an immune checkpoint on tumors.
FIG. 10 shows the inhibitory effect of PDE4 knockout on tumor metastasis.

### Detailed Description Of The Embodiments

The embodiments of the invention will be described in the following specific examples, and those skilled in the art can easily understand other advantages and effects of the invention from the disclosure in this specification.

### Definitions of Term

In the present application, the term "antagonist" generally refers to or includes partially or completely blocking, inhibiting, or neutralizing any molecule described herein, such as a protein For example, the molecules of the present application include, but are not limited to, CTLA-4, PD-1, PD-L1, PD-L2, LAG-3, TIM-3, galectin-9, CEACAM-1, BTLA, CD69, galectin-1, TIGIT, CD113, GPR56, VISTA, 2B4, CD48, GARP, PD1H, LAIR1, TIM-1, and/or TIM-4. Suitable antagonist molecules include, in particular, antagonist antibodies or antibody fragments, fragments or amino acid sequence variants of the molecules of the present application, peptides, antisense oligonucleotides, small organic molecules, and the like. The method of identifying an antagonist of a molecule of the present application comprises contacting cells expressing the molecule of the present application with a candidate antagonist molecule to detect one or more detectable changes in biological activity associated with the molecule of the present application. The antagonist can also be a peptide prepared by a rational design or by a phage display technique. When such a peptide is antagonized by itself, the peptide can also be fused to a cytotoxic agent to increase or enhance the antagonistic properties of the peptide.

In the present application, the term "variant" generally refers to a molecule in which the variant may comprise amino acid modifications (e.g., group substitutions, etc.) or the insertion, substitution, and/or deletion of one or more amino acids on the original protein sequence, while maintaining the function of the original sequence. For example, the variant may have better biological activity (function) than the original sequence. For example, the maintenance is not necessarily a complete maintenance. For example, the variant may substantially retain the functionality of the original sequence, e.g., at least 50%, 60%, 65%, 70%, 75%, 80%, 85%, 90%, 95%, 96%, 97%, 98%, or 99% functionality of the original sequence. For example, the amino acid sequence of the variant may be at least 50%, 70%, 75%, 80%, 85%, 90%, 95%, 96%, 97%, 98%, or 99% identical to the original amino acid sequence.

In the present application, the term "in combination" generally refers to the use of more than one treatment. The use of the term "in combination" does not limit the order in which the treatments are administered to a subject. The first treatment may be administered prior to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks), simultaneous or concomitant with, or subsequent to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks) administering the second treatment to the subject. Any additional treatment may be administered in any order with other additional treatment. For example, a PDE4-selective inhibitor of the present application may be administered in combination with one or more substances, such as an immune checkpoint inhibitor.

In the present application, the term "immune checkpoint inhibitor" generally refers to a molecule that, in whole or in part, reduces, inhibits, interferes with, or regulates one or more checkpoint proteins. Checkpoint proteins can regulate T cell activation or function. A number of checkpoint proteins are known to comprise, such as CTLA-4 and its ligands CD80 and CD86; and PD1 and its ligands PDL1 and PDL2. These proteins can be responsible for co-stimulation of the T cell response or inhibiting interactions. Immune checkpoint proteins can regulate and maintain their own tolerance and the duration and amplitude of the physiological immune response. Immune checkpoint inhibitors may include antibodies or polypeptides derived from antibodies.

In the present application, the term "container" generally refers to any vessel or device suitable for containing a medicament, e.g., kits, bottles, pouches, blisters, tubes, syringes, etc.

In the present application, the term "administration" generally refers to introducing the pharmaceutical combination into a subject's body by any introduction or delivery route. Any method known to those skilled in the art for contacting cells, organs, or tissues with the pharmaceutical combination can be employed, including, but not limited to, intraarterial, intranasal, intraperitoneal, intravenous, intramuscular, subcutaneous, transdermal or oral. The daily dose may be divided into one, two or more doses of suitable form to be administered at one, two or more times during a certain period of time.

In the present application, the term "subject" generally refers to human or non-human animals, including, but not limited to, cats, dogs, horses, pigs, cows, sheep, rabbits, mice, rats, or monkeys or the like.

In the present application, the term "sequential administration" generally means that when the active ingredients of the pharmaceutical combination are administered to the subject, the time interval between the time of administering the first active ingredient and the time of administering the last active ingredient is greater than 1 hour. For example, the active ingredients of the pharmaceutical combination may include a PDE4-selective inhibitor and/or an immune checkpoint inhibitor of the present application.

In the present application, the term "simultaneous" administration to the subject generally means that the active ingredients of the pharmaceutical combination are administered to the subject at the same time in the form of a composition/mixture, or when the active ingredients of the pharmaceutical combination are administered to the subject, the time interval between the time of administering the first active ingredient and the time of administering the last active ingredient does is not greater than 1 hour. For example, the active ingredients of the pharmaceutical combination may include a PDE4-selective inhibitor and/or an immune checkpoint inhibitor of the present application.

In the present application, the term "pharmaceutical combination" generally refers to a combination comprising at least two active ingredients/therapeutic agents. For example, each active ingredient/therapeutic agent may be individually prepared as a separate formulation (solid, liquid, gel, etc.), e.g., each active ingredient/therapeutic agent may be present in a different container, and can be simultaneously or separately prepared as a desired formulation with a suitable carrier as necessary; for example, each active ingredient/therapeutic agent may be from different sources (e.g., manufactured or sold by different vendors); for example, each active ingredient/therapeutic agent may form a pharmaceutical composition in admixture.

In the present application, the term "pharmaceutical composition" generally refers to a mixture comprising at least two active ingredients administered to a subject to treat a particular disease or disorder affecting the individual. It allows the active ingredient to be in an effective form and does not contain additional components that have unacceptable toxicity to the subject to which the composition is to be administered. Such a composition may be sterile or may contain a pharmaceutically acceptable carrier.

In the present application, the term "effective amount" or "effective dose" generally refers to an amount sufficient to achieve, or at least partially to achieve, the desired effect. The "therapeutically effective amount" or "therapeutically effective dose" of a drug or therapeutic agent is usually any amount of drug that, when used alone or in combination with another therapeutic agent, promotes the regression of a disease (as evidenced by a reduction in the severity of the symptoms of the disease, an increase in the frequency and duration of the asymptomatic period of the disease, or the prevention of damage or disability caused by suffering from the disease). The "prophylactically effective amount" or "prophylactically effective dose" of a drug generally refers to the amount of a drug that inhibits the development or recurrence of a disease when administered alone or in combination with another therapeutic agent to a subject at risk of disease progression or recurrence. The ability of a therapeutic or prophylactic agent to promote disease regression or inhibit disease progression or recurrence can be evaluated using a variety of methods known to those skilled in the art, such as predicting efficacy on human in human subjects during clinical trials and in animal model systems, or determining the activity of the agent in an *in vitro* assay.

In the present application, the term "carrier" generally refers to any substance other than the active ingredient. For example, the "carrier" of the present application may comprise a pharmaceutically acceptable carrier, and may not comprise a vector for inserting a DNA fragment, such as, pharmaceutically acceptable substances, compositions, or vehicles involved in carrying or transporting chemical agents. Examples include buffers, surfactants, stabilizers, preservatives, and absorption enhancers for enhancing bioavailability, liquid or solid fillers, diluents, excipients, solvents, encapsulation materials and/or other conventional solubilizers or dispersants. Each carrier must be "acceptable" in the sense that it is compatible with the other ingredients of the formulation and will not cause harm to the patient. For example, in the present application, a suitable carrier is selected depending on the form (as composition or each as a stand-alone formulation) in which each active ingredient (e.g., PDE4-selective inhibitor or immune checkpoint inhibitor) is present. For example, when each different active ingredient is independently present, it may be combined with a suitable carrier (i.e., the first carrier, the second carrier, or the third carrier described in the present application) respectively to form a formulation suitable for administration.

In the present application, the term "formulation," which may also be referred to as a pharmaceutical formulation, generally refers to a drug that can be provided to a subject for use in accordance with certain dosage form requirements in order to meet therapeutic or prophylactic needs. For example, the formulation may contain an active ingredient and a carrier.

In the present application, the term "prevention" generally refers to interventions taken in a healthy subject prior to the onset of symptoms and/or development of diseases to prevent the onset of a disease or disorder. It may also include prophylactically administering a combination to a patient in the prophase of an allergic disease to be treated. "Prevention" does not require 100% elimination of the likelihood of a disease or disorder, e.g., "prevention" usually means that the likelihood or occurrence of the disease or disorder is reduced in the presence of the administered combination.

In the present application, the term "treatment" generally refers to a clinical intervention for altering the natural course of the individual or cell being treated in a clinical pathological process. This may include improving disease status, eliminating lesions, or improving prognosis.

In the present application, the term "tumor" generally refers to a neoplasm or solid lesion formed by abnormal cell growth. In the present application, the tumor may be a solid tumor or a non-solid tumor. For example, a tangible mass that may be palpated by a clinical examination such as X-ray, CT scan, B-ultrasound, or palpation may be referred to as a solid tumor, while a tumor that cannot be seen or palpated by X-ray, CT scan, B-ultrasound and palpation, such as leukemia, may be referred to as a non-solid tumor.

In the present application, the term "metastatic tumor" generally refers to a tumor that has spread from its primary site to other sites in the body. Tumor cells can break away from the primary tumor, penetrate into lymph and blood vessels, circulate through the bloodstream and grow in distal lesions (metastases) in normal tissues elsewhere in the body. Metastases can be local or distal. Metastasis can be a continuous process, depending on how tumor cells detach from the primary tumor, spread through the bloodstream, and stop at distal sites. At the new site, the cells establish a blood supply and can grow to form life-threatening clumps. Both stimulatory and inhibitory molecular pathways within tumor cells can regulate this behavior, and interactions between tumor cells and host cells in distant sites can also be important.

In the present application, the term "CDR" also referred to as a "complementary determinant region" generally refers to a region in a variable domain of an antibody whose sequence is highly variable and/or forms a structurally defined loop. Typically, an antibody includes six CDRs; three in VH (HCDR1, HCDR2, HCDR3), and three in VL (LCDR1, LCDR2, LCDR3). For example, a naturally occurring camel antibody, consisting only of heavy chains, can also function normally and stably in the absence of light chains. See, e.g., Hamers-Casterman et al., Nature 363: 446-448 (1993); Sheriff et al, Nature Struct. Biol. 3: 733-736 (1996). CDRs of an antibody can be determined by a variety of coding systems, such as CCG, Kabat, Chothia, IMGT, Kabat/Chothia, etc. These coding systems are known in the art and can be found, e.g., at www.bioinf.org.uk/abs/index.html # kabatnum. For example, the amino acid sequence number of the antigen binding protein may be according to the IMGT numbering scheme (IMGT, the international ImMunoGeneTics information system@imgt.cines.fr; http://imgt.cines.fr; Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, DevComp Immunol 29: 185-203). For example, the CDRs of the antigen binding protein can be determined according to the Kabat numbering system (see, e.g., Kabat EA & Wu TT (1971) Ann NY AcadSci 190: 382-391 and Kabat EA et al., (1991) Sequences of Proteins of Immunological Interest, Fifth Edition, U.S. Department of Health and Human Services, NIH Publication No. 91-3242).

In the present application, the term "antibody" generally refers to an immunoglobulin or fragment thereof or a derivative thereof, encompassing any polypeptide including an antigen binding site, whether produced *in vitro* or *in vivo.* The term includes, but is not limited to, polyclonal, monoclonal, monospecific, multispecific, nonspecific, humanized, single-stranded, chimeric, synthetic, recombinant, hybrid, mutated, and transplanted antibodies. Unless otherwise modified by the term "intact," as in "intact antibody," the term "antibody" also includes antibody fragments, such as Fab, F(ab')₂, Fv, scFv, Fd, dAb, and other antibody fragments that maintain antigen binding function (e.g., specifically bind to PD-L1), for the purposes of the present application. Typically, such fragments should include an antigen binding domain. The basic 4-chain antibody unit is a heterotetrameric glycoprotein consisting of two identical light (L) chains and two identical heavy (H) chains. An IgM antibody consists of 5 basic heterotetrameric units and another polypeptide called a J chain, and contains 10 antigen binding sites, while an IgA antibody comprises 2-5 basic 4-chain units that can polymerize in conjunction with the J chain to form a multivalent combination. In the case of IgG, the 4-chain unit is typically about 150,000 Daltons. Each L chain is connected to the H chain by a covalent disulfide bond, while two H chains are connected to each other by one or more disulfide bonds depending on the isotype of the H chain. Each H and L chain also has regularly spaced intra-chain disulfide bridge bonds. Each H chain has a variable domain (VH) at the N-terminus, followed by three constant domains (CH) for each of the α and γ chains, and four CH domains for the µ and ε isotypes. Each L chain has a variable domain (VL) at the N-terminus and a constant domain at the other end. VL corresponds to VH, and CL corresponds to the first constant domain (CH1) of the heavy chain. Specific amino acid residues are thought to form an interface between the light chain and heavy chain variable domains. a VH and a VL pair together to form a single antigen binding site. For the structure and properties of different classes of antibodies, see, e.g., Basic and Clinical Immunology, 8th Edition, Daniel P. Sties, Abba I. Terr and Tristram G. Parsolw (eds.), Appleton & Lange, Norwalk, Conn., 1994, pp. 71 and chapter 6. L chains from any vertebrate species can be divided into one of two distinct types, called κ and λ, based on the amino acid sequence of their constant domain. According to the amino acid sequence of the heavy chain (CH) constant domain, immunoglobulins can be divided into different classes or isotypes. There are currently five classes of immunoglobulins: IgA, IgD, IgE, IgG, and IgM, with heavy chains designated α, δ, ε, γ, and µ, respectively.

In the present application, the term "antigen binding fragment" generally refers to one or more polypeptide fragments having the ability to specifically bind an antigen (e.g., PD-L1). In the present application, the antigen binding fragment may comprise a Fab, Fab', F(ab)₂, Fv fragment, F(ab')₂, scFv, di-scFv, and/or dAb.

In the present application, the term "variable region" generally refers to a region where certain segments of the variable domain may differ significantly in sequence between antibodies. The "variable region" in the light chain may comprise the light chain variable region VL; and the "variable region" in the heavy chain may comprise the heavy chain variable region VH. Variable domains mediate antigen binding and determine the specificity of a specific antibody to its particular antigen. However, variability is not evenly distributed across the entire variable domain. It is usually concentrated in three segments called hypervariable regions (CDRs or HVRs) in light and heavy chain variable domains. The more highly conserved part of the variable domain is called the framework region (FR). The variable domains of the natural heavy and light chains each comprise four FR regions, most of which are in a β-sheet configuration, linked by three CDRs, which form a circular connection, and in some cases form part of a β-sheet structure. The CDRs in each chain are held closely together by the FR region, and CDRs from the other chain together promote the formation of antigen binding sites of antibodies (see Kabat et al, Sequences of Immunological Interest, Fifth Edition, National Institute of Health, Bethesda, Md. (1991)).

In the present application, the term "Fab" generally refers to an antigen binding fragment of an antibody. As mentioned above, intact antibodies can be digested using papain. The antibody is digested by papain to produce two identical antigen binding fragments, the "Fab" fragment, and the remaining "Fc" fragment (i.e. Fc region, ibid.). The Fab fragment may consist of a complete L chain, a variable region of a heavy chain and a first constant region (CH1) of the H chain (VH).

In the present application, the term "Fab‴ or "Fab' fragment" generally refers to a monovalent antigen binding fragment of a human monoclonal antibody that is slightly larger than a Fab fragment. For example, the Fab' fragment may include all light chains, all heavy chain variable regions, and all or part of the first and second constant regions of the heavy chain. For example, the Fab' fragment may also include some or all of the 220-330 amino acid residues of the heavy chain.

In the present application, the term "(Fab')2" generally refers to an antibody fragment produced by digesting a intact antibody by a pepsin. The F(ab')2 fragment contains two Fab fragments held together by a disulfide bond and part of the hinge region. The F(ab')2 fragment have bivalent antigen binding activity and is capable of cross-linking antigens.

In the present application, the term "Fv" or "Fv fragment" generally refers to a monovalent antigen binding fragment of a human monoclonal antibody, including all or part of the heavy chain variable region and the light chain variable region, and lacking the heavy chain constant region and the light chain constant region. Heavy chain variable regions and light chain variable regions include, e.g., CDRs. For example, the Fv fragment comprises all or part of the amino-terminal variable regions of about 110 amino acids of the heavy chain and light chain.

In the present application, the term "scFv" generally refers to a fusion protein comprising at least one antibody fragment comprising a variable region of a light chain and at least one antibody fragment comprising a variable region of a heavy chain, wherein the light chain and heavy chain variable regions are contiguous (e.g., via a synthetic linker, e.g., a short flexible polypeptide linker) and capable of being expressed in the form of a single chain polypeptide, and wherein the scFv retains the specificity of the intact antibody from which it is derived. Unless otherwise specified, as used in the present application, the scFv may have the VL and VH variable regions described in any order (e.g., relative to the N-terminus and C-terminus of the polypeptide), and the scFv may comprise VL-linker-VH or may comprise VH-linker-VL.

In the present application, the term "dAb" generally refers to an antigen binding fragment having a VH domain and a VL domain, or a VH domain or a VL domain, with reference to, e.g., Ward et al. (Nature, 1989 Oct 12; 341 (6242): 544-6), Holt et al., Trends Biotechnol., 2003, 21 (11): 484-490; and for example, WO2006030220A1, WO2006003388A2, and other published patent applications of Domantis Ltd.

In the present application, the term "monoclonal antibody" generally refers to an antibody molecule preparation composed of a single molecule. Monoclonal antibodies are generally highly specific for a single antigenic site. Moreover, unlike conventional polyclonal antibody formulations, which typically have different antibodies against different determinants, each monoclonal antibody is directed against a single determinant on the antigen. In addition to their specificity, monoclonal antibodies have the advantage that they can be synthesized by hybridoma culture without being contaminated by other immunoglobulins. The modifier "monoclonal" denotes the characteristics of an antibody obtained from a substantially homogeneous population of antibodies and is not interpreted as requiring the production of an antibody by any particular method. For example, monoclonal antibodies used in the present application can be prepared in hybridoma cells or can be prepared by recombinant DNA methods.

In the present application, the term "chimeric antibody" generally refers to an antibody in which a variable region is derived from one species and a constant region is derived from another species. Typically, the variable region is derived from an antibody of an experimental animal, such as a rodent ("parental antibody"), and the constant region is derived from a human antibody, making the resulting chimeric antibody less likely to elicit an adverse immune response in a human individual than the parental (e.g. mouse-derived) antibody.

In the present application, the term "humanized antibody" generally refers to an antibody in which part or all of the amino acids outside the CDR region of a non-human antibody (e.g., a mouse antibody) are replaced by corresponding amino acids derived from a human immunoglobulin. Small additions, deletions, insertions, substitutions, or modifications of amino acids in the CDR region may also be permissible as long as they retain the ability of the antibody to bind to a particular antigen. The humanized antibody may optionally comprise at least a portion of a human immunoglobulin constant region. The "humanized antibody" retains antigen specificity similar to that of the original antibody. A "humanized" form of a non-human (e.g., mouse) antibody may comprise, at a minimum, a chimeric antibody derived from a sequence of a non-human immunoglobulin. In certain instances, CDR region residues in human immunoglobulins (receptor antibodies) can be replaced with CDR region residues of non-human species (donor antibodies), such as mice, rats, rabbits, or non-human primates, with the desired properties, affinity, and/or capabilities. In some cases, the FR region residues of the human immunoglobulin can be replaced with corresponding non-human residues. In addition, the humanized antibody may comprise amino acid modifications that are not present in the recipient antibody or in the donor antibody. These modifications may be made to further improve the performance of the antibody, such as binding affinity.

In the present application, the term "fully human antibody" generally refers to an antibody comprising only a human immunoglobulin protein sequence. If it is produced in mice, in mouse cells, or in hybridomas derived from mouse cells, the fully human antibody may contain a mouse sugar chain. Similarly, "mouse antibody" or "rat antibody" refers to an antibody that contain only a mouse or rat immunoglobulin sequence, respectively. Fully human antibodies can be generated in human body and transgenic animals with human immunoglobulin germline sequences by phage display or other molecular biological methods. Exemplary techniques that can be used to produce antibodies are described in U.S. Patent Nos. 6,150,584, 6,458,592 and 6,420,140. Other techniques, such as the use of libraries, are known in the art.

In the present application, the term "PD-1" or "PD1" generally refers to programmed cell death protein 1, a type I membrane protein of 288 amino acids, first described in 1992 (Ishida et al., EMBO J., 11 (1992), 3887-3895). PD-1 is a member of the extended CD28/CTLA-4T cell regulator family and has two ligands, PD-L1 (B7-H1, CD274) and PD-L2 (B7-DC, CD273). The structure of the protein consists of an extracellular IgV domain, followed by a transmembrane region and an intracellular tail. The intracellular tail contains two phosphorylation sites located in the tyrosine-based inhibitory motif of the immune receptor and the tyrosine-based conversion motif of the immune receptor, suggesting that PD-1 negatively regulates TCR signals. This is consistent with the binding of SHP-1 and SHP-2 phosphatases to the cytoplasmic tail of PD-1 after ligand binding. Although PD-1 is not expressed on naive T cells, it is upregulated after T cell receptor (TCR)-mediated activation and observed on both activated and depleted T cells (Agata et al. Int. Immunology 8 (1996), 765-772). These depleted T cells have a dysfunctional phenotype and cannot respond properly. Although PD-1 has a relatively wide expression pattern, its most important role is likely to be as a co-inhibitory receptor on T cells (Chinai et al. Trends in Pharmacological Sciences 36 (2015), 587-595). Current therapies thus focus on blocking the interaction of PD-1 with its ligands to enhance T cell response. The terms "programmed death 1," "programmed cell death 1," "protein PD-1," "PD-1," "PD1," "PDCD1," "hPD-1," and "hPD-I" are used interchangeably and include variants, isotypes, species homologues of human PD-1, and analogues that share at least one epitope with PD-1. The amino acid sequence of human PD1 can be shown in UniProt (www.uniprot.org) accession number Q15116.

In the present application, the term "PD-L1" or "PDL1" generally refers to programmed cell death 1 ligand 1, also referred to as B7 homolog 1, B7-H1, differentiation cluster 274, (3) 274, or CD274, which, upon binding to PD-1, down-regulates T cell activation and cytokine secretion. "PD-L1" includes any natural PD-L1 of any vertebrate origin, including mammals, such as primates (e.g., human and cynomolgus monkeys) and rodents (e.g., mice and rats). The term encompasses "full-length," unprocessed PD-L1, and any form of PD-L1 produced by cellular processing. PD-L1 may exist as a transmembrane protein or as a soluble protein. "PD-L1" includes complete PD-L1 and fragments thereof, and further includes functional variants, isoforms, species homologues, derivatives, analogues of PD-L1, and analogues that share at least one epitope with PD-1. The basic structure of PD-L1 consists of four domains: extracellular Ig-like V-type domain, Ig-like C2-type domain, transmembrane domain and cytoplasmic domain. Exemplary human PD-L1 amino acid sequences can be found under NCBI Accession No. NP_001254653 or UniProt Accession No. Q9NZQ7.

In the present application, the term "pembrolizumab" or "Pembrolizumab" generally refers to a PD-1 monoclonal antibody. The description of pembrolizumab can be found, for example, in U.S. Patent Nos. 8,354,509 and 8,900,587.

In the present application, the term "AMP-224" generally refers to an immune checkpoint inhibitor. For example, it can be a B7-DC Fc fusion protein. The description of AMP-224 can be found in U.S. Publication No. 2013/0017199 A.

In the present application, the term "BMS-936559" generally refers to an immune checkpoint inhibitor. For example, it may be an anti-PD-L1 antibody, and BMS-936559 may also be referred to as 12A4 or MDX-1105. The description of BMS-936559 can be found in U.S. Patent No. 7,943,743 or WO2013/173223A1.

In the present application, the term "MEDI4736" generally refers to an immune checkpoint inhibitor. For example, it can be an anti-PD-L1 antibody, and MEDI4736 can also be referred to as Durvalumab. The description of MEDI4736 can be found in Khleif (2013) In: Proceedings from the European Cancer Congress 2013; 27 September- 1 October 2013; Amsterdam, The Netherlands.

In the present application, the term "MPDL3280A" generally refers to an immune checkpoint inhibitor. For example, it may be an anti-PD-L1 antibody, and MPDL3280A may also be referred to as RG7446 or Atezolizumab. The description of MPDL3280A can be found in Herbst et al. (2013) J Clin Oncol 31 (suppl): 3000. Abstract, or U.S. Patent No. 8,217,149.

In the present application, the term "MSB0010718C" generally refers to an immune checkpoint inhibitor. For example, it can be an anti-PD-L1 antibody, and MSB0010718C can also be referred to as Bavencio or Avelumab. The description of MSB0010718C can be found in US 2014/0341917 A1.

In the present application, the term "PDE4" generally refers to a member of the PDE4 subfamily of the phosphodiesterase (PDEs) family capable of breaking the phosphodiester bond or a functional variant thereof. Phosphodiesterases (PDEs) have the function of hydrolyzing intracellular second messengers (cAMP, cyclic adenosine monophosphate or cGMP, cyclic guanosine monophosphate) and are capable of degrading intracellular cAMP or cGMP, thus terminating the biochemical effects mediated by these second messengers. PDEs are a large polygenic family, which can be divided into 12 families in mammals based on 1) amino acid sequence, 2) substrate specificity, 3) regulatory properties, 4) pharmacological properties and 5) tissue distribution, namely PDE1-PDE12. In PDE nomenclature, Arabic numerals are used to represent PDE family, followed by capital letters to represent genes in this family, and then the second Arabic numerals to represent spliced variants from a single gene (e.g., PDE1C3: family 1, gene C, spliced variant 3). The amino acid sequences of different PDEs in the same family may show significant differences, but they are functionally related. PDEs have different substrate specificities. For example, PDE4 is a cAMP selective hydrolase. In humans, PDE4 can be divided into four subtypes: PDE4A, 4B, 4C and 4D.

In the present application, the term "PDE4-selective inhibitors" (i.e., PDE4-selective inhibitors) generally refers to inhibitors that selectively act on the PDE4 subfamily in the PDE family. Among all members of the PDE family, PDE4-selective inhibitors mainly inhibit PDE4, but have little inhibitory effect on other PDE family members except PDE4. That is to say, PDE4-selective inhibitors are mainly relative to other members of PDE family, and it is possible that they have inhibitory effects on other molecules outside PDE family. PDE4-selective inhibitors can have inhibitory effects on one or more subtypes of the PDE4 family.

In the present application, the term "PDES-selective inhibitors" (i.e., PDE5-selective inhibitors) generally refers to inhibitors that selectively act on the PDE5 subfamily in the PDE family. For example, the PDE5-selective inhibitors may be Tadalafil (CAS #: 171596-29-5), Sildenafil citrate (CAS #: 171599-83-0), or Kuraridine (CAS #: 34981-25-4), and the like.

In the present application, the term "alkoxy" generally refers to a -OR group, wherein R may be an optionally substituted alkyl group.

In the present application, the term "alkyl" generally refers to a linear and branched saturated hydrocarbon group. Non-limiting examples thereof may include methyl, ethyl and linear and branched propyl and butyl groups.

In the present application, the term "halogen" refers to halogens of Group VIIA of the periodic table, such as F, Cl, Br and I.

In the present application, the term "amino" generally refers to a -NH group, wherein one or both of the hydrogen atoms can optionally be substituted, e.g., with alkyl, substituted alkyl, cycloalkyl, aryl, or heteroaryl.

In the present application, as is known to those skilled in the art, the terms "alkyl," "alkenyl," "cycloalkyl," and the like may be preceded by an identifier indicating the number of atoms present in the group in a particular case, e.g., C₁-C₄ alkyl, C₃-C₇ cycloalkoxy, C₁-C₄ alkylcarbonylamino, and the like, wherein a subscript number after "C" indicates the number of carbon atoms present in the group. For example, C₃ alkyl refers to an alkyl group having three carbon atoms (e.g., n-propyl, isopropyl); in C₁₋₁₀, members of the group may have any number of carbon atoms falling within the range of 1-4.

In the present application, the terms "comprising" or "including" generally mean including explicitly specified features, but not excluding other elements. In some cases, "comprising" or "including" also covers the case of "being" or "consisting of".

In the present application, the term "about" generally refers to a variation in the range of 0.5%-10% above or below a specified value, such as a variation in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

In the present application, as is known to those skilled in the art, the terms "alkyl," "alkenyl," "cycloalkyl," and the like may be preceded by an identifier indicating the number of atoms present in the group in a particular case, e.g., C₁-C₄ alkyl, C₃-C₇ cycloalkoxy, C₁-C₄ alkylcarbonylamino, and the like, wherein a subscript number after "C" indicates the number of carbon atoms present in the group. For example, C₃ alkyl refers to an alkyl group having three carbon atoms (e.g., n-propyl, isopropyl); in C₁₋₁₀, members of the group may have any number of carbon atoms falling within the range of 1-10.

### Detailed Description Of Invention

In one aspect, the present application provides a pharmaceutical combination, which may comprise:
a) an effective amount of an immune checkpoint inhibitor; and
b) an effective amount of a PDE4-selective inhibitor. For example, the PDE4-selective inhibitor of the present application may comprise a substance that reduces the expression of a gene encoding PDE4.

In another aspect, the present application also provides use of an immune checkpoint inhibitor and a PDE4-selective inhibitor in preparation of a medicament for preventing and/or treating a tumor. For example, the PDE4-selective inhibitor of the present application may comprise a substance that reduces the expression of a gene encoding PDE4.

In another aspect, the present application also provides use of a PDE4-selective inhibitor in preparation of a medicament for preventing and/or treating metastatic colon cancer. For example, the PDE4-selective inhibitor of the present application may comprise a substance that reduces the expression of a gene encoding PDE4. In another aspect, the present application also provides use of a PDE4-selective inhibitor in preparation of a medicament for preventing and/or treating metastatic colon cancer and/or metastatic breast cancer.

In another aspect, the present application also provides a method for preventing and/or treating a tumor, which may comprise administering to a subject in need thereof:
a) an effective amount of an immune checkpoint inhibitor; and
b) an effective amount of a PDE4-selective inhibitor.

In another aspect, the present application also provides a method for preventing and/or treating metastatic colon cancer, which may comprise administering to a subject in need thereof an effective amount of a PDE4-selective inhibitor.

In another aspect, the present application also provides an immune checkpoint inhibitor and a PDE4-selective inhibitor, which may be used to prevent and/or treat a tumor.

In another aspect, the present application also provides a PDE4-selective inhibitor, which can be used to prevent and/or treat metastatic colon cancer.

In another aspect, the present application also provides a kit, which may comprise a pharmaceutical combination of the present application.

In another aspect, the present application also provides a kit, which may comprise a PDE4-selective inhibitor of the present application.

In one aspect, the present application provides a pharmaceutical combination, which may comprise:
a) an effective amount of an immune checkpoint inhibitor; and
b) an effective amount of a compound of Formula I, a pharmaceutically acceptable salt thereof, or an N-oxide of pyridine or a salt thereof: 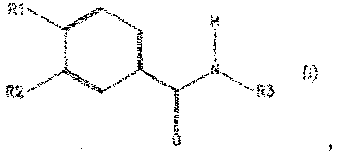 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
   R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In another aspect, the present application also provides use of an immune checkpoint inhibitor and a compound of Formula I, a pharmaceutically acceptable salt thereof, or an N-oxide of pyridine or a salt thereof: 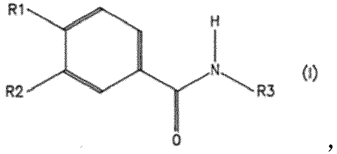 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 is C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen, in preparation of a medicament for preventing and/or treating a tumor.

In another aspect, the present application also provides use of a compound of Formula I, a pharmaceutically acceptable salt thereof, or an N-oxide of pyridine or a salt thereof: 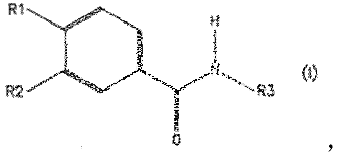 in preparation of a medicament for preventing and/or treating metastatic colon cancer, wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In another aspect, the present application also provides a method for preventing and/or treating a tumor, which may comprise administering to a subject in need thereof:
a) an effective amount of an immune checkpoint inhibitor; and
b) an effective amount of a compound of Formula I, a pharmaceutically acceptable salt thereof, or an N-oxide of pyridine or a salt thereof: 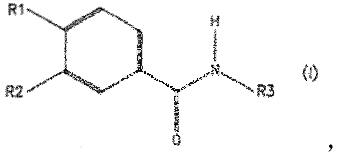 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
   R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In another aspect, the present application also provides a method for preventing and/or treating metastatic colon cancer, which may comprise administering to a subject in need thereof an effective amount of a compound of Formula I, a pharmaceutically acceptable salt thereof, or an N-oxide of pyridine or a salt thereof: 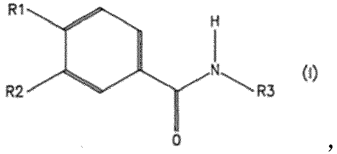 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In anoth er aspect, the present application also provides an immune checkpoint inhibitor and a compound of Formula I, a pharmaceutically acceptable salt thereof, or an N-oxide of pyridine or a salt thereof: 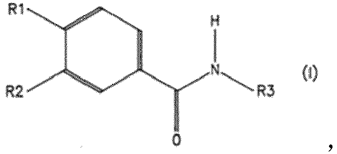 that can be used to prevent and/or treat tumors, wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In another aspect, the present application also provides a compound of Formula I, a pharmaceutically acceptable salt thereof, or an N-oxide of pyridine or a salt thereof: 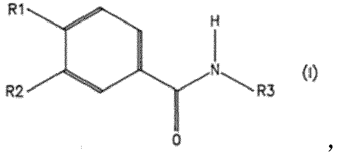 that can be used to prevent and/or treat metastatic colon cancer, wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In another aspect, the present application also provides a kit, which may comprise a pharmaceutical combination of the present application.

In another aspect, the present application also provides a kit, which may comprise a compound of Formula I of the present application, a pharmaceutically acceptable salt thereof, or an N-oxide of pyridine or a salt thereof: 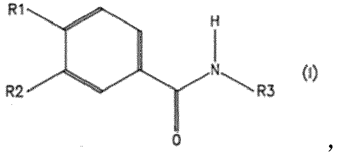 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

### Pharmaceutical combination

In one aspect, the pharmaceutical combination of the present application can comprise: prophylactically and/or therapeutically effective amounts of one or more PDE4-selective inhibitors.

In one aspect, the pharmaceutical combination of the present application can comprise: a) a prophylactically and/or therapeutically effective amount of an immune checkpoint inhibitor; and b) a prophylactically and/or therapeutically effective amount of a PDE4-selective inhibitor.

In one aspect, the pharmaceutical combination of the present application can comprise: prophylactically and/or therapeutically effective amounts of one or more compounds of Formula I, pharmaceutically acceptable salts thereof, or N-oxides of pyridine or salts thereof: 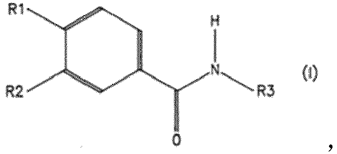 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In one aspect, the pharmaceutical combination of the present application can comprise: a) a prophylactically and/or therapeutically effective amount of an immune checkpoint inhibitor; and b) prophylactically and/or therapeutically effective amounts of one or more compounds of Formula I, pharmaceutically acceptable salts thereof, or N-oxides of pyridine or salts thereof: 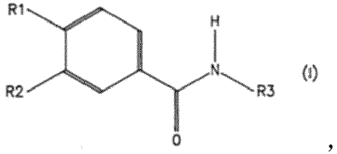 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In one aspect, the pharmaceutical composition of the present application can comprise: a) an antagonist of one or more proteins selected from the group consisting of CTLA-4, PD-1, PD-L1, PD-L2, LAG-3, TIM-3, galectin-9, CEACAM-1, BTLA, CD69, galectin-1, TIGIT, CD113, GPR56, VISTA, 2B4, CD48, GARP, PD1H, LAIR1, TIM-1, and TIM-4; and b) prophylactically and/or therapeutically effective amounts of one or more compounds of Formula I, pharmaceutically acceptable salts thereof, or N-oxides of pyridine, or salts thereof: 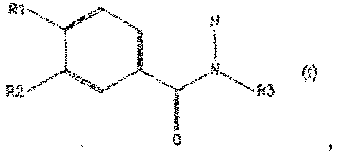 wherein R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

In one aspect, the pharmaceutical composition of the present application can comprise: a) an antibody comprising a HCDR3, or an antigen binding fragment thereof, and the HCDR3 comprises an amino acid sequence set forth in any one of SEQ ID NOs: 3, 13, 23, and 33; and b) prophylactically and/or therapeutically effective amounts of one or more compounds of Formula I, pharmaceutically acceptable salts thereof, or N-oxides of pyridine or salts thereof: 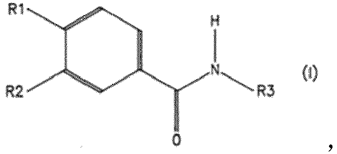 wherein R1 may be C₁-C₄-alkoxy fully or partially substituted with fluorine,
R2 may be Cs-Cs-cycloalkylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In one aspect, the pharmaceutical composition of the present application may comprise: a) an antibody comprising the following heavy chain and light chain, the heavy chain comprise a HCDR1 having an amino acid sequence set forth in any one of SEQ ID NOs: 1, 11, 21, and 31, a HCDR2 having an amino acid sequence set forth in any one of SEQ ID NOs: 2, 12, 22, and 32, and a HCDR3 having an amino acid sequence set forth in any one of SEQ ID NOs: 3, 13, 23, and 33, and the light chain comprises an LCDR1 having an amino acid sequence set forth in any one of SEQ ID NOs: 4, 14, 24, and 34, an LCDR2 having an amino acid sequence set forth in any one of SEQ ID NOs: 5, 15, 25, and 35, and an LCDR3 having an amino acid sequence set forth in any one of SEQ ID NOs: 6, 16, 26, and 36; and b) effective amounts of one or more compounds of Formula I, pharmaceutically acceptable salts thereof, or N-oxides of pyridine or salts thereof: 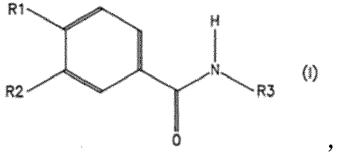 wherein R1 may be difluoromethoxy,
R2 may be cyclopropylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

In one aspect, the pharmaceutical composition of the present application may comprise: a) an antibody comprising the following heavy chain and light chain, the heavy chain comprises a VH of an amino acid sequence set forth in any one of SEQ ID NOs: 7, 17, 27 and 37, and the light chain comprises a VL of an amino acid sequence set forth in any one of SEQ ID NOs: 8, 18, 28 and 38; and b) effective amounts of one or more compounds of Formula I, pharmaceutically acceptable salts thereof, or N-oxides of pyridine or salts thereof: 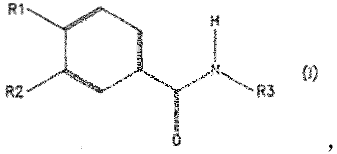 wherein R1 may be difluoromethoxy, R2 may be cyclopropylmethoxy, and R3 may be 3,5-dichloropyridin-4-yl.

In one aspect, the pharmaceutical composition of the present application can comprise: a) an antibody selected from the group consisting of nivolumab, pembrolizumab, BMS-936559, MPDL3280A, MEDI4736, AMP-224, and MSB0010718C; b) Roflumilast, or a derivative thereof, wherein the derivative of Roflumilast of the present application comprises Benzanilide, 3-hydroxy-N-(3,5-dichloropyridin-4-yl)-4-methoxybenzamide, and/or 4-Methylbenzanilide.

In the present application, the PDE4-selective inhibitor of the present application may include substances capable of at least partially disrupting or hindering the activity of one or more members of the PDE4 subfamily.

For example, members of the PDE4 subfamily may include PDE4A, PDE4B, PDE4C, and/or PDE4D.

For example, the PDE4-selective inhibitor of the present application may include substances that at least partially disrupt or hinder the activity of PDE4A, PDE4B, PDE4C, or PDE4D.

For example, the PDE4-selective inhibitor of the present application may include substances that at least partially disrupt or hinder the activity of PDE4C and PDE4D. For example, the PDE4-selective inhibitor of the present application may include substances that at least partially disrupt or hinder the activity of PDE4B and PDE4C. For example, the PDE4-selective inhibitor of the present application may include substances that at least partially disrupt or hinder the activity of PDE4B and PDE4D. For example, the PDE4-selective inhibitor of the present application may include substances that at least partially disrupt or hinder the activity of PDE4A and PDE4B. For example, the PDE4-selective inhibitor of the present application may include substances that at least partially disrupt or hinder the activity of PDE4A and PDE4C. For example, the PDE4-selective inhibitor of the present application may include substances that at least partially disrupt or hinder the activity of PDE4A and PDE4D.

For example, the PDE4-selective inhibitor of the present application may include substances that at least partially disrupt or hinder the activity of PDE4A, PDE4B, and PDE4C. For example, the PDE4-selective inhibitor of the present application may include substances that at least partially disrupt or hinder the activity of PDE4A, PDE4C, and PDE4D. For example, the PDE4-selective inhibitor of the present application may include substances that at least partially disrupt or hinder the activity of PDE4A, PDE4B, and PDE4D. For example, the PDE4-selective inhibitor of the present application may include substances that at least partially disrupt or hinder the activity of PDE4B, PDE4C, and PDE4D.

For example, the PDE4-selective inhibitor of the present application may include substances that at least partially disrupt or hinder the activity of PDE4A, PDE4B, PDE4C, and PDE4C.

For example, at least partial disruption or hindrance in the present application may include at least 30%, 40%, 50%, 55%, 60%, 65%, 70%, 75%, 80%, 85%, 90%, 95% disruption or hindrance.

For example, the PDE4-selective inhibitor of the present application may substantially not disrupt or hinder the activity of other members of the PDEs family (other than PDE4). For example, substantially no disruption or hindrance in the present application may include up to 30%, 25%, 20%, 18%, 15%, 13%, 10%, 8%, 5%, 3%, 1% disruption or hindrance.

For example, other members of the PDEs family of the present application may include members of the PDE1 subfamily, members of the PDE2 subfamily, members of the PDE3 subfamily, members of the PDE5 subfamily, members of the PDE6 subfamily, members of the PDE7 subfamily, members of the PDE8 subfamily, members of the PDE9 subfamily, members of the PDE10 subfamily, members of the PDE11 subfamily, and members of the PDE12 subfamily.

For example, the activity of the present application may include the activity of hydrolyzing cAMP and/or cGMP. For example, the activity of hydrolyzing cAMP and/or cGMP of the present application may be determined by any method known to those skilled in the art.

For example, the PDE4-selective inhibitor of the present application may comprise Apremilast (Otezla);
for example, the PDE4-selective inhibitor of the present application may comprise Crisaborole (Eucrisa);
for example, the PDE4-selective inhibitor of the present application may comprise Drotaverine;
for example, the PDE4-selective inhibitor of the present application may comprise CC-1088 (CAS #: 467421-06-3, 3-(1,3-dioxoisoindolin-2-yl)-3-phenylpropanamide, which can be found in K. Dredge (May 2005). "CC-1088 Celgene". Current Opinion in Investigational Drugs. 6 (5): 513-7. PMID 15912966);
for example, the PDE4-selective inhibitor of the present application may comprise BPN14770 (CAS #: 1606974-33-7, Benzeneacetic acid, 4-[[2-(3-chlorophenyl)-6-(trifluoromethyl)-4-pyridinyl] methyl]-, which can be found in Ricciarelli R, et al. Memory-enhancing effects of GEBR-32a, a new PDE4D inhibitor holding promise for the treatment of Alzheimer's disease. Sci Rep. 2017 Apr 12; 7: 46320.);
for example, the PDE4-selective inhibitor of the present application may comprise GSK356278 (CAS #: 720704-34-7, 5-[5-[(2,4-dimethyl-5-thiazolyl) methyl]-1,3,4-oxadiazol-2-yl]-1-ethyl-N-(tetrahydro-2H-pyran-4-yl)-1H-pyrazolo[3,4-b]pyridin-4-amine, available at doi.org/10.1124/jpet. 114.214155);
for example, the PDE4-selective inhibitor of the present application may comprise HT-0712 (CAS #: 617720-02-2, 2-Piperidinone, 5-[3-(cyclopentyloxy)-4-Methoxyphenyl]-3-[(3-Methylphenyl) Methyl]-, (3S,5S)-, which can be found in U.S. Patent No. 20040224316);
for example, the PDE4-selective inhibitor of the present application may comprise TAK-648 (available at doi: 10.1111/cts. 12436);
for example, the PDE4-selective inhibitor of the present application may comprise Zembrin (an extract of Sceletium tortuosum, whose active ingredient can be mesembrenone, mesembrenol, mesembranol, and/or mesembrine, available at doi: 10.1038/npp.2013.183);
for example, the PDE4-selective inhibitor of the present application may comprise ASP9831 (available at doi.org/10.1016/j.cgh.2014.01.040);
for example, the PDE4-selective inhibitor of the present application may comprise Etazolate (CAS #: 51022-77-6, ethyl 1-ethyl-4-(2-propan-2-ylidenehydrazinyl) Pyrazolo[3,4-b]pyridine-5-carboxylate, available at doi.org/10.1016/j.neuroscience.2014.01.008);
for example, the PDE4-selective inhibitor of the present application may comprise Piclamilast (CAS #: 144035-83-6, 3-cyclopentyloxy-N-(3,5-dichloropyridin-4-yl)-4-methoxybenzamide, available at doi.org/10.1124/jpet.102.047407);
for example, the PDE4-selective inhibitor of the present application may comprise Rolipram (CAS #: 61413-54-5, 4-(3-cyclopentyloxy-4-methoxyphenyl)pyrrolidin-2-one, available at doi.org/10.1073/pnas.0402595101);
for example, the PDE4-selective inhibitor of the present application may comprise Cilomilast (CAS #: 153259-65-5, 4-cyano-4-(3-cyclopentyloxy-4-methoxyphenyl)cyclohexane-1-carboxylic acid, available at doi.org/10.1164/rccm.200212-1490OC);
for example, the PDE4-selective inhibitor of the present application may comprise GSK256066 (available at doi.org/10.1016/j.pupt.2013.05.004);
for example, the PDE4-selective inhibitor of the present application may comprise AWD 12-281 (CAS #: 257892-33-4, N-(3,5-dichloro-4-pyridinyl)-2-[1-(4-fluorobenzyl)-5-hydroxy-1H-indol-3-yl]-2-oxoacetamide, available at doi.org/10.1124/jpet.103.053942);
for example, the PDE4-selective inhibitor of the present application may comprise Quinolyl oxazole (which can be found in U.S. Patent No. 7511062);
for example, the PDE4-selective inhibitor of the present application may comprise DC-TA-46 (CAS #: 109577-83-5, N-benzyl-6-chloro-2-piperazin-1-yl-4-pyrrolidin-1-ylpteridin-7-amine, available at doi.org/10.1210/en.2004-0562);
for example, the PDE4-selective inhibitor of the present application may comprise Filaminast (CAS #: 141184-34-1, [(E)-1-(3-cyclopentyloxy-4-methoxyphenyl) ethylideneamino] carbamate, available at doi.org/10.1517/13543784.8.9.1301); and/or
for example, the PDE4-selective inhibitor of the present application may comprise Glaucine (CAS #: 475-81-0, (6aS)-1,2,9,10-tetramethoxy-6-methyl-5,6,6a,7-tetrahydro-4H-dibenzo[de, g]quinoline, available at doi.org/10.1038/sj.bjp.0702702).

In one aspect, the pharmaceutical composition of the present application may comprise: a) an antibody comprising the following heacy chain and light chain, the heavy chain comprise a HCDR1 of an amino acid sequence set forth in any one of SEQ ID NOs: 1, 11, 21, and 31, a HCDR2 of an amino acid sequence set forth in any one of SEQ ID NOs: 2, 12, 22, and 32, and a HCDR3 of an amino acid sequence set forth in any one of SEQ ID NOs: 3, 13, 23, and 33, and the light chain comprises an LCDR1 of an amino acid sequence set forth in any one of SEQ ID NOs: 4, 14, 24, and 34, an LCDR2 of an amino acid sequence set forth in any one of SEQ ID NOs: 5, 15, 25, and 35, and an LCDR3 of an amino acid sequence set forth in any one of SEQ ID NOs: 6, 16, 26, and 36; and b) a compound of Formula I or a pharmaceutically acceptable salt thereof: 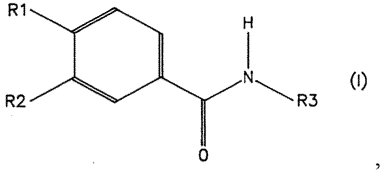 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be methoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₃-C₇-cycloalkoxy, C₃-C₇-cycloalkylmethoxy or benzyloxy,
R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be C₃-C₇-cycloalkylmethoxy or benzyloxy, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy, C₃-C₅-cycloalkoxy, Cs-Cs-cycloalkylmethoxy, benzyloxy or C₁-C₄-alkoxy fully or partially substituted with fluorine,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy, C₃-C₅-cycloalkoxy, Cs-Cs-cycloalkylmethoxy, benzyloxy or C₁-C₄-alkoxy fully or partially substituted with fluorine,
R2 may be methoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₃-C₅-cycloalkoxy, Cs-Cs-cycloalkylmethoxy or benzyloxy,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be C₁-C₄-alkoxy, C₃-C₅-cycloalkoxy, Cs-Cs-cycloalkylmethoxy, benzyloxy or C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be Cs-Cs-cycloalkylmethoxy or benzyloxy, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy, C₃-C₅-cycloalkoxy, Cs-Cs-cycloalkylmethoxy, benzyloxy or C₁-C₄-alkoxy fully or partially substituted with fluorine,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy, C₃-C₅-cycloalkoxy, C₃-C₅-cycloalkylmethoxy, benzyloxy or C₁-C₄-alkoxy fully or partially substituted with fluorine,
R2 may be methoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₃-C₅-cycloalkoxy, Cs-Cs-cycloalkylmethoxy or benzyloxy,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be C₁-C₄-alkoxy, C₃-C₅-cycloalkoxy, Cs-Cs-cycloalkylmethoxy, benzyloxy or C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be Cs-Cs-cycloalkylmethoxy or benzyloxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be methoxy, ethoxy, isopropoxy, isobutoxy, cyclopentoxy, cyclopropylmethoxy, cyclobutylmethoxy, difluoromethoxy or 2,2,2-trifluoroethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be methoxy, cyclopropylmethoxy, cyclobutylmethoxy or difluoromethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be methoxy, n-propoxy, n-butoxy, cyclopropylmethoxy or 2,2,2-trifluoroethoxy,
R2 may be difluoromethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be cyclopropylmethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₃-C₇-cycloalkoxy or C₃-C₇-cycloalkylmethoxy, and R2 may be C₁-C₄-alkoxy fully or partially substituted with fluorine; or R1 may be C₁-C₄-alkoxy fully or partially substituted with fluorine, and R2 may be C₃-C₇-cycloalkylmethoxy; and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C₄-alkyl, or C₁-C₄-alkoxy, R33 may be hydrogen, halogen, C₁-C4-alkyl, or C₁-C₄-alkoxy, R34 may be hydroxy, halogen, carboxyl, C₁-C₄-alkyl, C₁-C₄-alkoxy, or C₁-C₄-alkoxycarbonyl, R35 may be hydrogen, halogen, amino, or C₁-C₄-alkyl, R36 may be hydrogen or halogen, and R37 may be hydrogen or halogen;
for example, wherein
R1 may be C₃-C₇-cycloalkoxy or C₃-C₇-cycloalkylmethoxy,
R2 may be C₁-C₄-alkoxy fully or partially substituted with fluorine, and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C4-alkyl, or C₁-C₄-alkoxy, R33 may be hydrogen, halogen, C₁-C4-alkyl, or C₁-C₄-alkoxy, R34 may be hydroxy, halogen, carboxyl, C1-C4-alkyl, C₁-C₄-alkoxy, or C₁-C₄-alkoxycarbonyl, R35 may be hydrogen, halogen, amino, or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be C₃-C₇-cycloalkylmethoxy, and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C4-alkyl, or C₁-C₄-alkoxy, R33 may be hydrogen, halogen, C₁-C4-alkyl, or C₁-C₄-alkoxy, R34 may be hydroxy, halogen, carboxyl, C1-C4-alkyl, C₁-C₄-alkoxy, or C₁-C₄-alkoxycarbonyl, R35 may be hydrogen, halogen, or C₁-C₄-alkyl, R36 may be hydrogen or halogen, and R37 may be hydrogen or halogen;
for example, wherein
R1 may be C₃-C₅-cycloalkoxy or Cs-Cs-cycloalkylmethoxy,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be Cs-Cs-cycloalkylmethoxy, and
R3 may be phenyl, pyridinyl, phenyl substituted with R31, R32 and R33 or pyridinyl substituted with R34, R35, R36 and R37, wherein R31 may be halogen, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₃-C₅-cycloalkoxy, Cs-Cs-cycloalkylmethoxy or benzyloxy,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be Cs-Cs-cycloalkylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be cyclopropylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein R1 may be difluoromethoxy, R2 may be cyclopropylmethoxy, and R3 may be 3,5-dichloropyridin-4-yl.

For example, the PDE4-selective inhibitor of the present application may comprise Roflumilast or a derivative thereof.

For example, the derivative of Roflumilast of the present application may comprise benzamide and/or bezafibrate.

For example, a ratio of an effective amount of the PDE4-selective inhibitor of the present application to an effective amount of the immune checkpoint inhibitor of the present application may be from about 1:10 to about 1:5000. For example, a ratio of an effective amount of the PDE4-selective inhibitor of the present application to an effective amount of the immune checkpoint inhibitor of the present application may be from about 9:1 to about 1:4.

For example, a ratio of an effective amount of the PDE4-selective inhibitor of the present application to an effective amount of the immune checkpoint inhibitor of the present application may be from about 9:1 to about 1:4, e.g., a ratio of an effective amount of the PDE4-selective inhibitor of the present application to an effective amount of the immune checkpoint inhibitor of the present application may be from about 8:1 to about 1:4, from about 7:1 to about 1:4, from about 6:1 to about 1:4, from about 5:1 to about 1:4, from about 4:1 to about 1:4, from about 3:1 to about 1:4, from about 2:1 to about 1:4, from about 1:1 to about 1:4, from about 1:2 to about 1:4, or from about 1:3 to about 1:4, e.g., a ratio of an effective amount of the PDE4-selective inhibitor of the present application to an effective amount of the immune checkpoint inhibitor of the present application may be from 9:1 to about 1:3, from about 8:1 to about 1:3, from about 7:1 to about 1:3, from about 6:1 to about 1:3, from about 5:1 to about 1:3, from about 4:1 to about 1:3, from about 3:1 to about 1:3, from about 2:1 to about 1:3, from about 1:1 to about 1:3, or from about 1:2 to about 1:3, e.g., a ratio of an effective amount of the PDE4-selective inhibitor of the present application to an effective amount of the immune checkpoint inhibitor of the present application may be from 9:1 to about 1:2, from about 8:1 to about 1:2, from about 7:1 to about 1:2, from about 6:1 to about 1:2, from about 5:1 to about 1:2, from about 4:1 to about 1:2, from about 3:1 to about 1:2, from about 2:1 to about 1:2, or from about 1:1 to about 1:2, e.g., a ratio of an effective amount of the PDE4-selective inhibitor of the present application to an effective amount of the immune checkpoint inhibitor of the present application may be from 9:1 to about 1:1, from about 8:1 to about 1:1, from about 7:1 to about 1:1, from about 6:1 to about 1:1, from about 5:1 to about 1:1, from about 4:1 to about 1:1, from about 3:1 to about 1:1, or from about 2:1 to about 1:1, e.g., a ratio of an effective amount of the PDE4-selective inhibitor of the present application to an effective amount of the immune checkpoint inhibitor of the present application may be from 9:1 to about 3:1, from about 8:1 to about 3:1, from about 7:1 to about 3:1, from about 6:1 to about 3:1, from about 5:1 to about 3:1, or from about 4:1 to about 3:1, e.g., a ratio of an effective amount of the PDE4-selective inhibitor of the present application to an effective amount of the immune checkpoint inhibitor of the present application may be from 9:1 to about 6:1, from about 8:1 to about 6:1, or from about 7:1 to about 6:1, e.g., a ratio of an effective amount of the PDE4-selective inhibitor of the present application to an effective amount of the immune checkpoint inhibitor of the present application may be from 9:1 to about 7:1, or from about 8:1 to about 7:1, For example, a ratio of an effective amount of the PDE4-selective inhibitor of the present application to an effective amount of the immune checkpoint inhibitor of the present application may be from 9:1 to about 8:1.

For example, the ratio of effective amounts of the present application may include the molar ratio of effective amounts and/or the mass ratio of effective amounts.

For example, the mass ratio of the PDE4-selective inhibitor of the present application to the immune checkpoint inhibitor of the present application may be from about 5000:1 to about 1:5000.

For example, the mass ratio of the PDE4-selective inhibitor of the present application to the immune checkpoint inhibitor of the present application may be from about 9:1 to about 1:4, e.g., the mass ratio of the PDE4-selective inhibitor of the present application to the immune checkpoint inhibitor of the present application may be from about 8:1 to about 1:4, from about 7:1 to about 1:4, from about 6:1 to about 1:4, from about 5:1 to about 1:4, from about 4:1 to about 1:4, from about 3:1 to about 1:4, from about 2:1 to about 1:4, from about 1:1 to about 1:4, from about 1:2 to about 1:4, or from about 1:3 to about 1:4, e.g., the mass ratio of the PDE4-selective inhibitor of the present application to the immune checkpoint inhibitor of the present application may be from 9:1 to about 1:3, from about 8:1 to about 1:3, from about 7:1 to about 1:3, from about 6:1 to about 1:3, from about 5:1 to about 1:3, from about 4:1 to about 1:3, from about 3:1 to about 1:3, from about 2:1 to about 1:3, from about 1:1 to about 1:3, or from about 1:2 to about 1:3, e.g., the mass ratio of the PDE4-selective inhibitor of the present application to the immune checkpoint inhibitor of the present application may be from 9:1 to about 1:2, from about 8:1 to about 1:2, from about 7:1 to about 1:2, from about 6:1 to about 1:2, from about 5:1 to about 1:2, from about 4:1 to about 1:2, from about 3:1 to about 1:2, from about 2:1 to about 1:2, or from about 1:1 to about 1:2, e.g., the mass ratio of the PDE4-selective inhibitor of the present application to the immune checkpoint inhibitor of the present application may be from 9:1 to about 1:1, from about 8:1 to about 1:1, from about 7:1 to about 1:1, from about 6:1 to about 1:1, from about 5:1 to about 1:1, from about 4:1 to about 1:1, from about 3:1 to about 1:1, or from about 2:1 to about 1:1, e.g., the mass ratio of the PDE4-selective inhibitor of the present application to the immune checkpoint inhibitor of the present application may be from 9:1 to about 3:1, from about 8:1 to about 3:1, from about 7:1 to about 3:1, from about 6:1 to about 3:1, from about 5:1 to about 3:1, or from about 4:1 to about 3:1, e.g., the mass ratio of the PDE4-selective inhibitor of the present application to the immune checkpoint inhibitor of the present application may be from 9:1 to about 6:1, from about 8:1 to about 6:1, or from about 7:1 to about 6:1, e.g., the mass ratio of the PDE4-selective inhibitor of the present application to the immune checkpoint inhibitor of the present application may be from 9:1 to about 7:1, or from about 8:1 to about 7:1, e.g., the mass ratio of the PDE4-selective inhibitor of the present application to the immune checkpoint inhibitor of the present application may be from 9:1 to about 8:1.

For example, the PDE4-selective inhibitor of the present application and the immune checkpoint inhibitor of the present application may each be from different sources, e.g., those manufactured or sold by different suppliers. The immune checkpoint inhibitor of the present application as prepared, manufactured or sold is not necessarily single-component or pure, and any one can be within the scope of the present application as long as it comprises a compound capable of releasing the immune checkpoint inhibitor or solvates, hydrates, salts thereof *in vivo* or *in vitro,* and/or an analogue of the immune checkpoint inhibitor of the present application (e.g., those in which one or more amino acids of the immune checkpoint inhibitor of the present application are replaced by other amino acid(s), while the immune checkpoint inhibitor of the present application maintains substantially the stable activity of the immune checkpoint inhibitor of the present application *in vitro* or *in vivo*)*.* Likewise, the PDE4-selective inhibitor as prepared, manufactured or sold is not necessarily single-component or pure, and any one can be within the scope of the present application as long as it comprises a compound capable of releasing any one or more of the PDE4-selective inhibitors of the present application or solvates, hydrates, salts thereof *in vivo* or *in vitro,* and/or any one or more analogues of the PDE4-selective inhibitors of the present application (e.g., those which comprise modifications or substitutions of groups, and maintain substantially the activity of any PDE4-selective inhibitor *in vitro* or *in vivo*)*.*

For example, the PDE4-selective inhibitor of the present application and the immune checkpoint inhibitor of the present application are present in different containers, respectively. For example, the PDE4-selective inhibitor of the present application and the immune checkpoint inhibitor of the present application may be present in the same container.

For example, the PDE4-selective inhibitor of the present application and the immune checkpoint inhibitor of the present application may or may not be set depending on the ratio of their effective amounts.

For example, the PDE4-selective inhibitor of the present application may be present in one or more containers, such as 2 or more, 3 or more, 4 or more, 5 or more, 6 or more, 7 or more, 8 or more, 9 or more, 10 or more, 11 or more, or 12 or more.

For example, the immune checkpoint inhibitor of the present application may be present in one or more containers, such as 2 or more, 3 or more, 4 or more, 5 or more, 6 or more, 7 or more, 8 or more, 9 or more, 10 or more, 11 or more, or 12 or more.
for example, containers of the present application may include medicine boxes, kits, medicine bottles, pouches, blisters, tubes, syringes, and the like; for example, medicine bottles of the present application may include sealed glass ampoules, test tubes, vials, flasks, bottles, and the like; for example, containers of the present application may be made of glass, an organic polymer such as polycarbonate and polystyrene, or ceramic, metal, composite film, cellophane, foil-lined packaging material such as aluminum or alloy, and any other suitable material that can generally be used to hold the reagents, and the like.

For example, the PDE4-selective inhibitor of the present application and the immune checkpoint inhibitor of the present application may each be present in different containers, and may be concurrently or separately formulated with suitable carriers as needed.

For example, the PDE4-selective inhibitor of the present application and the immune checkpoint inhibitor of the present application may be mixed to formulate a suitable formulation.

For example, the PDE4-selective inhibitor of the present application and the immune checkpoint inhibitor of the present application may each be formulated into different suitable formulations.

For example, the PDE4-selective inhibitor of the present application and the immune checkpoint inhibitor of the present application may each be present in different containers in the form of a formulation.

For example, the pharmaceutical combination of the present application may comprise a first formulation and a second formulation, wherein the first formulation of the present application may comprise the immune checkpoint inhibitor of the present application and a first pharmaceutically acceptable carrier, and the second formulation of the present application may comprise the PDE4-selective inhibitor of the present application and a second pharmaceutically acceptable carrier.

For example, the first formulation of the present application and the second formulation of the present application may be in the same dosage form or different dosage forms.

For example, the first formulation of the present application and the second formulation of the present application may all be in any of the dosage forms such as pills, powders, tablets, granules, gels, hard capsules, soft capsules, syrups, mixtures, distillate formulas, injections, aerosols, ointments, pellicles, suppositories, drops, and the like, or any different combination thereof. For example, both the first formulation of the present application and the second formulation of the present application may be injections or tablets. For example, the first formulation of the present application may be a tablet, and the second formulation may be an injection. For example, the first formulation of the present application may be an injection, and the second formulation may be a tablet. For example, the first formulation of the present application may be a tablet, and the second formulation of the present application may be a granule.

For example, the first formulation of the present application and the second formulation of the present application may be in the form of formulations suitable for the same mode of administration. For example, the first formulation of the present application and the second formulation of the present application may both be tablets, granules, hard capsules, soft capsules, or syrups suitable for oral administration. For example, both the first formulation of the present application and the second formulation of the present application may be injections suitable for injection.

For example, the first formulation of the present application and the second formulation of the present application may be in the form of formulations suitable for different modes of administration. For example, the first formulation of the present application and the second formulation of the present application may be tablets, granules, hard capsules, soft capsules, or syrups suitable for oral administration. For example, the first formulation of the present application may be an injection suitable for injection, and the second formulation of the present application may be a tablet, a granule, a hard capsule, a soft capsule, or a syrup suitable for oral administration. For example, the first formulation of the present application may be an injection suitable for injection, and the second formulation of the present application may be a sustained release formulation suitable for embedding sustained release encapsulated microcapsules and the like.

For example, the pharmaceutical combination of the present application may comprise a pharmaceutical composition, e.g., the pharmaceutical composition of the present application may comprise a PDE4-selective inhibitor of the present application and an immune checkpoint inhibitor of the present application.

For example, the PDE4-selective inhibitor of the present application may be contained in the pharmaceutical composition of the present application in an amount of about 20% (w/w) to about 90% (w/w).

For example, the PDE4-selective inhibitor of the present application may be contained in the pharmaceutical composition of the present application in an amount of about 20% (w/w) to about 90% (w/w), about 30% (w/w) to about 90% (w/w), about 40% (w/w) to about 90% (w/w), about 50% (w/w) to about 90% (w/w), about 60% (w/w) to about 90% (w/w), about 70% (w/w) to about 90% (w/w), about 80% (w/w) to about 90% (w/w), about 85% (w/w) to about 90% (w/w), or about 89% (w/w) to about 90% (w/w), e.g., the PDE4-selective inhibitor of the present application may be contained in the pharmaceutical composition of the present application in an amount of about 20% (w/w) to about 80% (w/w), about 30% (w/w) to about 80% (w/w), about 40% (w/w) to about 80% (w/w), about 50% (w/w) to about 80% (w/w), about 60% (w/w) to about 80% (w/w), or about 70% (w/w) to about 80% (w/w), e.g., the PDE4-selective inhibitor of the present application may be contained in the pharmaceutical composition of the present application in an amount of about 20% (w/w) to about 70% (w/w), about 30% (w/w) to about 70% (w/w), about 40% (w/w) to about 70% (w/w), about 50% (w/w) to about 70% (w/w), or about 60% (w/w) to about 70% (w/w), e.g., the PDE4-selective inhibitor of the present application may be contained in the pharmaceutical composition of the present application in an amount of about 20% (w/w) to about 60% (w/w), about 30% (w/w) to about 60% (w/w), about 40% (w/w) to about 60% (w/w), or about 50% (w/w) to about 60% (w/w), e.g., the PDE4-selective inhibitor of the present application may be contained in the pharmaceutical composition of the present application in an amount of about 20% (w/w) to about 50% (w/w), about 30% (w/w) to about 50% (w/w), or about 40% (w/w) to about 50% (w/w), e.g., the PDE4-selective inhibitor of the present application may be contained in the pharmaceutical composition of the present application in an amount of about 20% to about 40% (w/w), or about 30% to about 40% (w/w), e.g., the PDE4-selective inhibitor of the present application may be contained in the pharmaceutical composition of the present application in an amount of about 20% (w/w) to about 30% (w/w), or about 25% (w/w) to about 30% (w/w).

For example, the immune checkpoint inhibitor of the present application may be contained in the pharmaceutical composition of the present application in an amount of about 10% (w/w) to about 80% (w/w).

For example, the immune checkpoint inhibitor of the present application may be contained in the pharmaceutical composition of the present application in an amount of about 10% (w/w) to about 80% (w/w), about 20% (w/w) to about 80% (w/w), about 30% (w/w) to about 80% (w/w), about 40% (w/w) to about 80% (w/w), about 50% (w/w) to about 80% (w/w), about 60% (w/w) to about 80% (w/w), about 70% (w/w) to about 80% (w/w), or about 75% (w/w) to about 80% (w/w), e.g., the immune checkpoint inhibitor of the present application may be contained in the pharmaceutical composition of the present application in an amount of about 10% (w/w) to about 70% (w/w), about 20% (w/w) to about 70% (w/w), about 30% (w/w) to about 70% (w/w), about 40% (w/w) to about 70% (w/w), about 50% (w/w) to about 70% (w/w), or about 60% (w/w) to about 70% (w/w), e.g., the immune checkpoint inhibitor of the present application may be contained in the pharmaceutical composition of the present application in an amount of about 10% (w/w) to about 60% (w/w), about 20% (w/w) to about 60% (w/w), about 30% (w/w) to about 60% (w/w), about 40% (w/w) to about 60% (w/w), or about 50% (w/w) to about 60% (w/w), e.g., the immune checkpoint inhibitor of the present application may be contained in the pharmaceutical composition of the present application in an amount of about 10% (w/w) to about 50% (w/w), about 20% (w/w) to about 50% (w/w), about 30% (w/w) to about 50% (w/w), or about 40% (w/w) to about 50% (w/w), e.g., the immune checkpoint inhibitor of the present application may be contained in the pharmaceutical composition of the present application in an amount of about 10% (w/w) to about 40% (w/w), about 20% (w/w) to about 40% (w/w), or about 30% (w/w) to about 40% (w/w), e.g., the immune checkpoint inhibitor of the present application may be contained in the pharmaceutical composition of the present application in an amount of about 10% (w/w) to about 30% (w/w), or about 20% (w/w) to about 30% (w/w), e.g., the immune checkpoint inhibitor of the present application may be contained in the pharmaceutical composition of the present application in an amount of about 10% (w/w) to about 20% (w/w), or about 15% (w/w) to about 20% (w/w).

### Kit

A kit of the present application may include a pharmaceutical combination of the present application. The pharmaceutical combination of the present application may be provided in the form of a kit, wherein the different components of the pharmaceutical combination may be packaged in different containers, may be mixed prior to administration, or may be administered separately without mixing.

For example, the separate packaging may be for long-term storage without losing the functions of the active constituents.

For example, the formulation contained in the kit may be present in any type of container in which components of the formulation remain active for a long time, are not adsorbed by the container material, and are not easily deteriorated, e.g., sealed glass ampoules. For example, ampoules may be made of glass, an organic polymer such as polycarbonate and polystyrene, ceramic, metal, or any other suitable material that can generally be used to hold the reagent, and the like. Examples of other suitable containers include simple bottles made of substances similar to ampoules, and packaging materials lined with foil such as aluminum or alloys. Other containers may include test tubes, vials, flasks, bottles, syringes, or the like. The container has a sterile access port for a bottle or the like, which may have a stopper which can be penetrated by a hypodermic needle.

For example, the kit may also comprise a buffer packaged in the presence of a neutral and non-reactive gas such as nitrogen.

For example, the kit may also include an auxiliary administration device, such as a measuring cup and a measuring spoon suitable for oral administration, such as a syringe suitable for injection, an infusion tube, an infusion needle, and the like.

For example, instructions for use may also be attached to the kit. Instructions for use of the kit consisting of the pharmaceutical combination may be printed on paper or other material and/or supplied in the form of electrically or electromagnetically readable media such as Floppy disks, CD-ROMs, DVD-ROMs, Zip disks, videotapes, audiotapes, and the like. Detailed instructions for use may physically be attached to the kit or posted on a website designated by the manufacturer or distributor of the kit or notified by email.

For example, the pharmaceutical combination provided by the present application may be described in the instruction for use of the present application.

For example, the use or method provided by the present application may be described in the instruction for use of the present application.

### Uses and Methods

The present application provides use of the combination of the PDE4-selective inhibitor of the present application and the immune checkpoint inhibitor of the present application in preparation of a medicament for preventing and/or treating a tumor. The present application also provides use of the PDE4-selective inhibitor in preparation of a medicament for preventing and/or treating a tumor.

The present application also provides a method for preventing and/or treating a tumor, which may comprise administering to a subject in need thereof:
a) an effective amount of an immune checkpoint inhibitor; and
b) an effective amount of a PDE4-selective inhibitor.

For example, the tumor may include a solid tumor and/or a non-solid tumor.

For example, the solid tumor may be selected from the group consisting of bowel cancer, breast cancer, and melanoma.

For example, the tumor comprises colon cancer and/or melanoma.

For example, the tumor comprises a metastatic tumor.

For example, the tumor comprises a tumor with liver metastasis.

For example, the tumor comprises colon cancer with liver metastasis.

In one aspect, the PDE4-selective inhibitor, pharmaceutical combination or kit of the present application may be used to prevent and/or treat a tumor, which may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., the PDE4-selective inhibitor, pharmaceutical combination or kit of the present application may be used to prevent and/or treat a tumor, which may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

In one aspect, the PDE4-selective inhibitor, pharmaceutical combination, or kit of the present application may be used to prevent and/or treat a metastatic tumor, which may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., the PDE4-selective inhibitor, pharmaceutical combination, or kit of the present application may be used to prevent and/or treat a metastatic tumor, which may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

In another aspect, the present application provides a method for preventing and/or treating a tumor, which may comprise administering to a subject in need thereof the PDE4-selective inhibitor, pharmaceutical combination or kit of the present application for preventing and/or treating the tumor, which may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., which may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

In another aspect, the present application provides a method for preventing and/or treating a metastatic tumor, which may comprise administering to a subject in need thereof the PDE4-selective inhibitor, pharmaceutical combination, or kit of the present application for preventing and/or treating a metastatic tumor, which may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., which may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

In another aspect, the present application provides use of the PDE4-selective inhibitor, pharmaceutical combination, or kit of the present application in preparation of a medicament for preventing and/or treating a tumor, which may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., which may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

In another aspect, the present application provides use of the PDE4-selective inhibitor, pharmaceutical combination, or kit of the present application in preparation of a medicament for preventing and/or treating a tumor, the metastatic tumor may be reduced in volume by about 1%, about 2%, about 3%, about 4%, about 5%, about 10%, about 20%, about 30%, about 40%, about 50%, about 60%, about 70%, about 80%, about 90%, or about 100% compared to a blank control group, e.g., the metastatic tumor may be reduced in volume by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, or about 1000-fold compared to a blank control group.

For example, the PDE4-selective inhibitor of the present application and the immune checkpoint inhibitor of the present application may be configured to be administered to a subject simultaneously.

For example, the PDE4-selective inhibitor of the present application and the immune checkpoint inhibitor of the present application may be administered to the subject simultaneously.

For example, the simultaneous administrations to a subject may include a time interval of no more than 1 hour between the administration of the PDE4-selective inhibitor of the present application to the subject and the administration of the immune checkpoint inhibitor of the present application to the subject. For example, the time interval may be 60 minutes, 55 minutes, 50 minutes, 45 minutes, 40 minutes, 35 minutes, 30 minutes, 25 minutes, 20 minutes, 15 minutes, 10 minutes, 8 minutes, 5 minutes, 3 minutes, 2 minutes, 1 minute, or may be administered together in a mixed form.

For example, the PDE4-selective inhibitor of the present application and the immune checkpoint inhibitor of the present application may be configured to be administered separately to a subject.

For example, the PDE4-selective inhibitor of the present application and the immune checkpoint inhibitor of the present application may be administered to the subject sequentially.

For example, the PDE4-selective inhibitor of the present application and the immune checkpoint inhibitor of the present application may each be configured to administer to a subject in any order, which may include administering the PDE4-selective inhibitor of the present application, followed by the immune checkpoint inhibitor of the present application, or administering the immune checkpoint inhibitor of the present application, followed by the PDE4-selective inhibitor of the present application. For example, the separate administrations to a subject may include a time interval of more than 1 hour between the administration of the PDE4-selective inhibitor of the present application to the subject and the administration of the immune checkpoint inhibitor of the present application to the subject. For example, the time interval may be 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer.

For example, the sequential administrations to a subject may include a time interval of more than 1 hour between the administration of the PDE4-selective inhibitor of the present application to the subject and the administration of the immune checkpoint inhibitor of the present application to the subject. For example, the time interval may be 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer.

For example, administering the PDE4-selective inhibitor of the present application may include administering once.

For example, administering the PDE4-selective inhibitor of the present application may include administering successively the PDE4-selective inhibitor two or more times, e.g., three or more times, four or more times, five or more times, six or more times, seven or more times, eight or more times, nine or more times, ten or more times, and then administering the immune checkpoint inhibitor of the present application.

For example, administering the immune checkpoint inhibitor of the present application may include administering once.

For example, administering the immune checkpoint inhibitor of the present application may include administering successively the immune checkpoint inhibitor two or more times, e.g., three or more times, four or more times, five or more times, six or more times, seven or more times, eight or more times, nine or more times, ten or more times, and then administering the PDE4-selective inhibitor of the present application.

For example, the medicament of the present application may be configured such that the PDE4-selective inhibitor of the present application and the immune checkpoint inhibitor of the present application are administered at a mass ratio from about 1:5 to about 1:5000.

For example, the PDE4-selective inhibitor of the present application and the immune checkpoint inhibitor of the present application can be administered at a mass ratio from about 1:5 to about 1:5000.

For example, the medicament may be configured such that the PDE4-selective inhibitor of the present application and the immune checkpoint inhibitor of the present application are administered at a ratio of effective amounts from about 8:1 to about 1:4, from about 7:1 to about 1:4, from about 6:1 to about 1:4, from about 5:1 to about 1:4, from about 4:1 to about 1:4, from about 3:1 to about 1:4, from about 2:1 to about 1:4, from about 1:1 to about 1:4, from about 1:2 to about 1:4, or from about 1:3 to about 1:4, e.g., the PDE4-selective inhibitor of the present application and the immune checkpoint inhibitor of the present application may be administered at a ratio of effective amounts from 9:1 to about 1:3, from about 8:1 to about 1:3, from about 7:1 to about 1:3, from about 6:1 to about 1:3, from about 5:1 to about 1:3, from about 4:1 to about 1:3, from about 3:1 to about 1:3, from about 2:1 to about 1:3, from about 1:1 to about 1:3, or from about 1:2 to about 1:3, e.g., the PDE4-selective inhibitor of the present application and the immune checkpoint inhibitor of the present application may be administered at a ratio of effective amounts from about 9:1 to about 1:2, from about 8:1 to about 1:2, from about 7:1 to about 1:2, from about 6:1 to about 1:2, from about 5:1 to about 1:2, from about 4:1 to about 1:2, from about 3:1 to about 1:2, from about 2:1 to about 1:2, or from about 1:1 to about 1:2, e.g., the PDE4-selective inhibitor of the present application and the immune checkpoint inhibitor of the present application may be administered at a ratio of effective amounts from about 9:1 to about 1:1, from about 8:1 to about 1:1, from about 7:1 to about 1:1, from about 6:1 to about 1:1, from about 5:1 to about 1:1, from about 4:1 to an effective amount ratio of from about 1:1, from about 3:1 to about 1:1, or from about 2:1 to about 1:1, e.g., the PDE4-selective inhibitor of the present application and the immune checkpoint inhibitor of the present application may be administered at a ratio of effective amounts from about 9:1 to about 3:1, from about 8:1 to about 3:1, from about 7:1 to about 3:1, from about 6:1 to about 3:1, from about 5:1 to about 3:1, or from about 4:1 to about 3:1, e.g., the PDE4-selective inhibitor of the present application and the immune checkpoint inhibitor of the present application may be administered at a ratio of effective amounts from 9:1 to about 6:1, from about 8:1 to about 6:1, or from about 7:1 to about 6:1, e.g., the PDE4-selective inhibitor of the present application and the immune checkpoint inhibitor of the present application may be administered at a ratio of effective amounts from 9:1 to about 7:1, or from about 8:1 to about 7:1, e.g., the PDE4-selective inhibitor of the present application and the immune checkpoint inhibitor of the present application may be administered at a ratio of effective amounts from 9:1 to about 8:1.

For example, the medicament may be configured such that the PDE4-selective inhibitor of the present application and the immune checkpoint inhibitor of the present application are administered at a ratio of effective amounts from about 1:5 to about 1:5000.

For example, the PDE4-selective inhibitor of the present application and the immune checkpoint inhibitor of the present application can be administered at a mass ratio of about 1:5 to about 1:5000.

For example, the medicament may be configured such that the PDE4-selective inhibitor of the present application and the immune checkpoint inhibitor of the present application are administered at a mass ratio from about 8:1 to about 1:4, from about 7:1 to about 1:4, from about 6:1 to about 1:4, from about 5:1 to about 1:4, from about 4:1 to about 1:4, from about 3:1 to about 1:4, from about 2:1 to about 1:4, from about 1:1 to about 1:4, from about 1:2 to about 1:4, or from about 1:3 to about 1:4, e.g., the PDE4-selective inhibitor of the present application and the immune checkpoint inhibitor of the present application may be administered at a mass ratio from 9:1 to about 1:3, from about 8:1 to about 1:3, from about 7:1 to about 1:3, from about 6:1 to about 1:3, from about 5:1 to about 1:3, from about 4:1 to about 1:3, from about 3:1 to about 1:3, from about 2:1 to about 1:3, from about 1:1 to about 1:3, or from about 1:2 to about 1:3, e.g., the PDE4-selective inhibitor of the present application and the immune checkpoint inhibitor of the present application may be administered at a mass ratio from about 9:1 to about 1:2, from about 8:1 to about 1:2, from about 7:1 to about 1:2, from about 6:1 to about 1:2, from about 5:1 to about 1:2, from about 4:1 to about 1:2, from about 3:1 to about 1:2, from about 2:1 to about 1:2, or from about 1:1 to about 1:2, e.g., the PDE4-selective inhibitor of the present application and the immune checkpoint inhibitor of the present application may be administered at a mass ratio from about 9:1 to about 1:1, from about 8:1 to about 1:1, from about 7:1 to about 1:1, from about 6:1 to about 1:1, from about 5:1 to about 1:1, from about 4:1 to an effective amount ratio of from about 1:1, from about 3:1 to about 1:1, or from about 2:1 to about 1:1, e.g., the PDE4-selective inhibitor of the present application and the immune checkpoint inhibitor of the present application may be administered at a mass ratio from about 9:1 to about 3:1, from about 8:1 to about 3:1, from about 7:1 to about 3:1, from about 6:1 to about 3:1, from about 5:1 to about 3:1, or from about 4:1 to about 3:1, e.g., the PDE4-selective inhibitor of the present application and the immune checkpoint inhibitor of the present application may be administered at a mass ratio from 9:1 to about 6:1, from about 8:1 to about 6:1, or from about 7:1 to about 6:1, e.g., the PDE4-selective inhibitor of the present application and the immune checkpoint inhibitor of the present application may be administered at a mass ratio from 9:1 to about 7:1, or from about 8:1 to about 7:1, e.g., the PDE4-selective inhibitor of the present application and the immune checkpoint inhibitor of the present application may be administered at a mass ratio from 9:1 to about 8:1.

For example, the ratio of effective amounts may include the molar ratio of effective amounts and/or the mass ratio of effective amounts.

For example, the administration may include intravenous, arterial, subcutaneous, muscular, intradermal, intracavitary, intratumoral, peritumoral administration, and the like. For example, the administration can be oral administration, application, and the like. The mode of administration may depend on a variety of factors, e.g., in order to obtain an effective concentration at the site where the tumor is located, the medicament may be administered in a variety of routes, such as selective arterial perfusion, intracavitary perfusion, intraperitoneal or intracutaneous administration, and intravertebral administration. For example, the administration can be topical administration, e.g., through selective arterial, intratumoral, peritumoral injection or placement, e.g., in the form of intratumoral, peritumoral, or intratumoral slow release.

### PDE4-selective Inhibitor

In the present application, said PDE4-selective inhibitor may include substances capable of at least partially disrupting or hindering the activity of one or more members of the PDE4 subfamily.

For example, said PDE4 subfamily members may include PDE4A, PDE4B, PDE4C, and/or PDE4D.

For example, said PDE4-selective inhibitor may include substances that at least partially disrupt or hinder the activity of PDE4A, PDE4B, PDE4C, or PDE4D.

For example, said PDE4-selective inhibitor may include substances that at least partially disrupt or hinder the activity of PDE4C and PDE4D. For example, said PDE4-selective inhibitor may include substances that at least partially disrupt or hinder the activity of PDE4B and PDE4C. For example, said PDE4-selective inhibitor may include substances that at least partially disrupt or hinder the activity of PDE4B and PDE4D. For example, said PDE4-selective inhibitor may include substances that at least partially disrupt or hinder the activity of PDE4A and PDE4B. For example, said PDE4-selective inhibitor may include substances that at least partially disrupt or hinder the activity of PDE4A and PDE4C. For example, said PDE4-selective inhibitor may include substances that at least partially disrupt or hinder the activity of PDE4A and PDE4D.

For example, said PDE4-selective inhibitor can include substances that at least partially disrupt or hinder the activity of PDE4A, PDE4B, and PDE4C. For example, said PDE4-selective inhibitor can include substances that at least partially disrupt or hinder the activity of PDE4A, PDE4C, and PDE4D. For example, said PDE4-selective inhibitor can include substances that at least partially disrupt or hinder the activity of PDE4A, PDE4B, and PDE4D. For example, said PDE4-selective inhibitor can include substances that at least partially disrupt or hinder the activity of PDE4B, PDE4C, and PDE4D.

For example, said PDE4-selective inhibitors may include substances that at least partially disrupt or hinder the activity of PDE4A, PDE4B, PDE4C, and PDE4C.

For example, said at least partial disruption or hindrance may include at least 30%, 40%, 50%, 55%, 60%, 65%, 70%, 75%, 80%, 85%, 90%, 95% disruption or hindrance.

For example, said PDE4-selective inhibitor may substantially not disrupt or hinder the activity of other members of the PDEs family (other than PDE4). For example, said substantially no disruption or hindrance may include up to 30%, 25%, 20%, 18%, 15%, 13%, 10%, 8%, 5%, 3%, 1% disruption or hindrance.

For example, said other members of the PDEs family may include members of the PDE1 subfamily, members of the PDE2 subfamily, members of the PDE3 subfamily, members of the PDE5 subfamily, members of the PDE6 subfamily, members of the PDE7 subfamily, members of the PDE8 subfamily, members of the PDE9 subfamily, members of the PDE10 subfamily, members of the PDE11 subfamily, and members of the PDE12 subfamily.

For example, said activity may include the activity of hydrolyzing cAMP and/or cGMP. For example, said activity of hydrolyzing cAMP and/or cGMP may be determined by any method known to those skilled in the art.

For example, said PDE4-selective inhibitor may comprise Apremilast (Otezla), Crisaborole (Eucrisa), Drotaverine, CC-1088 (found in K. Dredge (May 2005). "CC-1088 Celgene". Current Opinion in Investigational Drugs. 6 (5): 513-7. PMID 15912966), BPN14770 (found in Ricciarelli R, et al. Memory-enhancing effects of GEBR-32a, a new PDE4D inhibitor holding promise for the treatment of Alzheimer's disease. Sci Rep. 2017 Apr 12; 7: 46320.), GSK356278 (available at doi.org/10.1124/jpet.114.214155), HT-0712 (found in U.S. Patent No. 20040224316), TAK-648 (available at doi: 10.1111/cts.12436), Zembrin (available at doi: 10.1038/npp.2013.183), ASP9831 (available at doi.org/10.1016/j.cgh.2014.01.040), Etazolate (available at doi.org/10.1016/j.neuroscience.2014.01.008), Piclamilast (available at doi.org/10.1124/jpet.102.047407), Rolipram (available at doi.org/10.1073/pnas.0402595101), Cilomilast (available at doi.org/10.1164/rccm.200212-1490OC), GSK256066 (available at doi.org/10.1016/j.pupt.2013.05.004), AWD 12-281 (available at doi.org/10.1124/jpet.103.053942), Quinolyl oxazole (found in U.S. Patent No. 7511062), DC-TA-46 (available at doi.org/10.1210/en.2004-0562), Filaminast (available at doi.org/10.1517/13543784.8.9.1301) and/or Glaucine (available at doi.org/10.1038/sj.bjp.0702702).

For example, the present application provides a compound of Formula I or a pharmaceutically acceptable salt thereof: 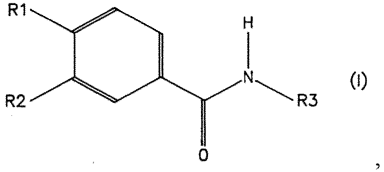 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be methoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₃-C₇-cycloalkoxy, C₃-C₇-cycloalkylmethoxy or benzyloxy,
R2 may be C₁-C₄-alkoxy fully or partially substituted with fluorine, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be C₃-C₇-cycloalkylmethoxy or benzyloxy, and
R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen;
for example, wherein
R1 may be C₁-C₄-alkoxy, Cs-Cs-cycloalkoxy, Cs-Cs-cycloalkylmethoxy, benzyloxy or C₁-C₄-alkoxy fully or partially substituted with fluorine,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy, Cs-Cs-cycloalkoxy, Cs-Cs-cycloalkylmethoxy, benzyloxy or C₁-C₄-alkoxy fully or partially substituted with fluorine,
R2 may be methoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be Cs-Cs-cycloalkoxy, Cs-Cs-cycloalkylmethoxy or benzyloxy,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be C₁-C₄-alkoxy, Cs-Cs-cycloalkoxy, Cs-Cs-cycloalkylmethoxy, benzyloxy or C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be Cs-Cs-cycloalkylmethoxy or benzyloxy, and
R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy, Cs-Cs-cycloalkoxy, Cs-Cs-cycloalkylmethoxy, benzyloxy or C₁-C₄-alkoxy fully or partially substituted with fluorine,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy, Cs-Cs-cycloalkoxy, Cs-Cs-cycloalkylmethoxy, benzyloxy or C₁-C₄-alkoxy fully or partially substituted with fluorine,
R2 may be methoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be Cs-Cs-cycloalkoxy, Cs-Cs-cycloalkylmethoxy or benzyloxy,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be C₁-C₄-alkoxy, Cs-Cs-cycloalkoxy, Cs-Cs-cycloalkylmethoxy, benzyloxy or C₁-C₄-alkoxy fully or partially substituted with fluorine, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be Cs-Cs-cycloalkylmethoxy or benzyloxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be methoxy, ethoxy, isopropoxy, isobutoxy, cyclopentoxy, cyclopropylmethoxy, cyclobutylmethoxy, difluoromethoxy or 2,2,2-trifluoroethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be methoxy, cyclopropylmethoxy, cyclobutylmethoxy or difluoromethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be methoxy, n-propoxy, n-butoxy, cyclopropylmethoxy or 2,2,2-trifluoroethoxy,
R2 may be difluoromethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be cyclopropylmethoxy, and
R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₃-C₇-cycloalkoxy or C₃-C₇-cycloalkylmethoxy, and R2 may be C₁-C₄-alkoxy fully or partially substituted with fluorine; or R1 may be C₁-C₄-alkoxy fully or partially substituted with fluorine, and R2 may be C₃-C₇-cycloalkylmethoxy; and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C₄-alkyl, or C₁-C₄-alkoxy, R33 may be hydrogen, halogen, C₁-C₄-alkyl, or C₁-C₄-alkoxy, R34 may be hydroxy, halogen, carboxyl, C1-C4-alkyl, C₁-C₄-alkoxy, or C₁-C₄-alkoxycarbonyl, R35 may be hydrogen, halogen, amino, or C₁-C₄-alkyl, R36 may be hydrogen or halogen, and R37 may be hydrogen or halogen;
for example, wherein
R1 may be C₃-C₇-cycloalkoxy or C₃-C₇-cycloalkylmethoxy,
R2 may be C₁-C₄-alkoxy fully or partially substituted with fluorine, and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C4-alkyl, or C₁-C₄-alkoxy, R33 may be hydrogen, halogen, C₁-C4-alkyl, or C₁-C₄-alkoxy, R34 may be hydroxy, halogen, carboxyl, C1-C4-alkyl, C₁-C₄-alkoxy, or C₁-C₄-alkoxycarbonyl, R35 may be hydrogen, halogen, amino, or C₁-C₄-alkyl, R36 may be hydrogen or halogen, and R37 may be hydrogen or halogen;
for example, wherein
R1 may be C₁-C₄-alkoxy fully or partially substituted with fluorine,
R2 may be C₃-C₇-cycloalkylmethoxy, and
R3 may be phenyl, pyridinyl, phenyl substituted with R31, R32, and R33, or pyridinyl substituted with R34, R35, R36, and R37, wherein: R31 may be hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 may be hydrogen, hydroxy, halogen, trifluoromethyl, C₁-C4-alkyl, or C₁-C₄-alkoxy, R33 may be hydrogen, halogen, C₁-C4-alkyl, or C₁-C₄-alkoxy, R34 may be hydroxy, halogen, carboxyl, C1-C4-alkyl, C₁-C₄-alkoxy, or C₁-C₄-alkoxycarbonyl, R35 may be hydrogen, halogen, or C₁-C₄-alkyl, R36 may be hydrogen or halogen, and R37 may be hydrogen or halogen;
for example, wherein
R1 may be C₃-C₅-cycloalkoxy or Cs-Cs-cycloalkylmethoxy,
R2 may be C₁-C₄-alkoxy fully or partially substituted with fluorine, and
R3 may be phenyl, pyridinyl, phenyl substituted with R31, R32 and R33 or pyridinyl substituted with R34, R35, R36 and R37, wherein R31 may be halogen, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be C₁-C₄-alkoxy fully or partially substituted with fluorine,
R2 may be Cs-Cs-cycloalkylmethoxy, and
R3 may be phenyl, pyridinyl, phenyl substituted with R31, R32 and R33 or pyridinyl substituted with R34, R35, R36 and R37, wherein R31 may be halogen, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen;
for example, wherein
R1 may be Cs-Cs-cycloalkoxy, Cs-Cs-cycloalkylmethoxy or benzyloxy,
R2 may be C₁-C₄-alkoxy fully or partially substituted with fluorine, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be C₁-C₄-alkoxy fully or partially substituted with fluorine,
R2 may be Cs-Cs-cycloalkylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein
R1 may be difluoromethoxy,
R2 may be cyclopropylmethoxy, and
R3 may be 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl;
for example, wherein R1 may be difluoromethoxy, R2 may be cyclopropylmethoxy, and R3 may be 3,5-dichloropyridin-4-yl.

For example, said PDE4-selective inhibitor or the present application may comprise a compound of Formula I or a pharmaceutically acceptable salt thereof: 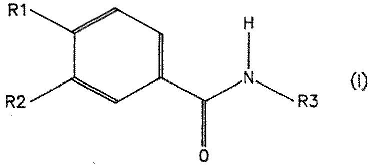
for example, wherein R1 may be C₁-C₄-alkoxy fully or partially substituted with fluorine, and R2 may be methoxy;
or, e.g., wherein R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and R2 may be methoxy fully or partially substituted with fluorine;
or, e.g., wherein R1 may be C₃-C₇-cycloalkoxy, C₃-C₇-cycloalkylmethoxy, or benzyloxy, and R2 may be C₁-C₄-alkoxy fully or partially substituted with fluorine;
or, e.g., wherein R1 may be C₁-C₄-alkoxy fully or partially substituted with fluorine, and R2 may be C₃-C₇-cycloalkylmethoxy or benzyloxy;
and, R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, said PDE4-selective inhibitor or the present application may comprise a compound of Formula I or a pharmaceutically acceptable salt thereof: 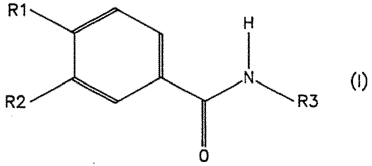
for example, wherein R1 may be C₁-C₄-alkoxy fully or partially substituted with fluorine, and R2 may be methoxy;
or, e.g., wherein R1 may be C₁-C₄-alkoxy, Cs-Cs-cycloalkoxy, Cs-Cs-cycloalkylmethoxy, benzyloxy, or C₁-C₄-alkoxy fully or partially substituted with fluorine, and R2 may be methoxy fully or partially substituted with fluorine;
or, e.g., wherein R1 may be Cs-Cs-cycloalkoxy, Cs-Cs-cycloalkylmethoxy, or benzyloxy, and R2 may be C₁-C₄-alkoxy fully or partially substituted with fluorine;
or, e.g., wherein R1 may be C₁-C₄-alkoxy fully or partially substituted with fluorine, and R2 may be Cs-Cs-cycloalkylmethoxy or benzyloxy;
and, R3 may be phenyl, pyridinyl, phenyl substituted with R31, R32 and R33 or pyridinyl substituted with R34, R35, R36 and R37, wherein R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, said PDE4-selective inhibitor or the present application may comprise a compound of Formula I or a pharmaceutically acceptable salt thereof: 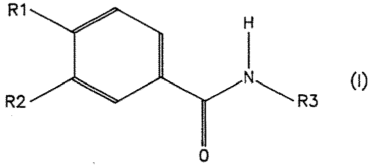
for example, wherein R1 may be C₁-C₄-alkoxy fully or partially substituted with fluorine, and R2 may be methoxy;
or, e.g., wherein R1 may be C₁-C₄-alkoxy, Cs-Cs-cycloalkoxy, Cs-Cs-cycloalkylmethoxy, benzyloxy, or C₁-C₄-alkoxy fully or partially substituted with fluorine, and R2 may be methoxy fully or partially substituted with fluorine;
or, e.g., wherein R1 may be Cs-Cs-cycloalkoxy, Cs-Cs-cycloalkylmethoxy, or benzyloxy, and R2 may be C₁-C₄-alkoxy fully or partially substituted with fluorine;
or, e.g., wherein R1 may be C₁-C₄-alkoxy fully or partially substituted with fluorine, and R2 may be Cs-Cs-cycloalkylmethoxy or benzyloxy;
and, R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3 -yl.

For example, C₁-C₆-alkoxy may be hexyl, isohexyl (2-methylpentyl), neohexyl (2,2-dimethylbutyl), pentyl, isopentyl (3-methylbutyl), neopentyl (2,2-dimethylpropyl), butyl, isobutyl, sec-butyl, tert-butyl, propyl, isopropyl, ethyl, or methyl.

For example, C₃-C₇-cycloalkoxy may be cyclopropoxy, cyclobutoxy, cyclopentoxy, cyclohexoxy, or cycloheptoxy.

For example, C₃-C₇-cycloalkylmethoxy may be cyclopropylmethoxy, cyclobutylmethoxy, cyclopentylmethoxy, cyclohexylmethoxy, or cycloheptylmethoxy.

For example, C₁-C₄-alkoxy fully or partially substituted with fluorine may be 1,2,2-trifluoroethoxy, 2,2,3,3,3-pentafluoropropoxy, perfluoroethoxy, 1,1,2,2-tetrafluoroethoxy, trifluoromethoxy, 2,2,2-trifluoroethoxy, or difluoromethoxy.

For example, halogen may refer to bromine, chlorine, or fluorine.

For example, C₁-C₄-alkyl may refer to a linear or branched alkyl group having 1 to 4 carbon atoms. Examples include butyl, isobutyl, sec-butyl, tert-butyl, propyl, isopropyl, ethyl, and methyl.

For example, C₁-C₄-alkoxy may be methoxy and ethoxy.

For example, C₁-C₄-alkoxycarbonyl may be methoxycarbonyl (CH₃O-CO-) and ethoxycarbonyl (CH₃CH₂O-CO-).

For example, C₁-C₄-alkylcarbonyl may refer to an acetyl group (CH₃CO-).

For example, C₁-C₄-alkylcarbonyloxy may be an acetic acid group (CH₃CO-O-).

For example, mono- or di-C₁-C₄-alkylamino may be methylamino, dimethylamino, or diethylamino.

For example, C₁-C₄-alkylcarbonylamino may be acetamido (-NH-CO-CH₃).

For example, phenyl substituted with R31, R32, and R33 may be a group selected from the group consisting of 2-acetylphenyl, 2-aminophenyl, 2-bromophenyl, 2-chlorophenyl, 2,3-dichlorophenyl, 2,4-dichlorophenyl, 4-diethylamino-2-methylphenyl, 4-bromo-2-trifluoromethylphenyl, 2-carboxyl-5-chlorophenyl, 3,5-dichloro-2-hydroxyphenyl, 2-bromo-4-carboxyl-5-hydroxyphenyl, 2,6-dichlorophenyl, 2,5-dichlorophenyl, 2,4,6-trichlorophenyl, 2,4,6-trifluorophenyl, 2,6-dibromophenyl, 2-cyanophenyl, 4-cyano-2-fluorophenyl, 2-fluorophenyl, 2,4-difluorophenyl, 2,6-difluorophenyl, 2-chloro-6-fluorophenyl, 2-hydroxyphenyl, 2-hydroxy-4-methoxyphenyl, 2,4-dihydroxyphenyl, 2-methoxyphenyl, 2,3-dimethoxyphenyl, 2,4-dimethoxyphenyl, 2,6-dimethoxyphenyl, 2-dimethylaminophenyl, 2-methylphenyl, 2-chloro-6-methylphenyl, 2,4-dimethylphenyl, 2,6-dimethylphenyl, 2,3-dimethylphenyl, 2-methoxycarbonylphenyl, 2-trifluoromethylphenyl, 2,6-dichloro-4-methoxyphenyl, 2,6-dichloro-4-cyanophenyl, 2,6-dichloro-4-aminophenyl, 2,6-dichloro-4-methoxycarbonylphenyl, 4-acetamido-2,6-dichlorophenyl and 2,6-dichloro-4-ethoxycarbonylphenyl.

For example, pyridinyl substituted with R34, R35, R36, and R37 may be a group selected from the group consisting of 3,5-dichloropyridin-4-yl, 2,6-diaminopyridin-3-yl, 4-aminopyridin-3-yl, 3-methylpyridin-2-yl, 4-methylpyridin-2-yl, 5-hydroxypyridin-2-yl, 4-chloropyridin-3-yl, 3-chloropyridin-2-yl, 3-chloropyridin-4-yl, 2-chloropyridin-3-yl, 2,3,5,6-tetrafluoropyridin-4-yl, 3,5-dichloro-2,6-difluoropyridin-4-yl, 3,5-dibromopyridin-2-yl, 3,5-dibromopyridin-4-yl, 3,5-dichloropyridin-4-yl, 2,6-dichloropyridin-3-yl, 3,5-dimethylpyridin-4-yl, 3-chloro-2,5,6-trifluoropyridin-4-yl and 2,3,5-trifluoropyridin-4-yl.

For example, said PDE4-selective inhibitor or the compound of the present application may comprise a compound of Formula I or a pharmaceutically acceptable salt thereof: 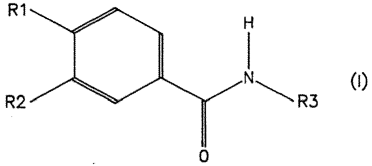 wherein one of R1 and R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 may be C₁-C₄-alkoxy fully or partially substituted with fluorine, and
R3 may be phenyl, pyridinyl, phenyl substituted with R31, R32, and R33, or pyridinyl substituted with R34, R35, R36, and R37, wherein:
R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄- alkylcarbonyloxy, amino, mono- or di-C₁-C₄-alkylamino, or C₁-C₄-alkylcarbonylamino,
R32 may be hydrogen, hydroxyl, halogen, amino, trifluoromethyl, C₁-C₄ alkyl or C₁-C₄ alkoxy,
R33 may be hydrogen, halogen, C₁-C₄ alkyl or C₁-C₄ alkoxy,
R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl,
R36 may be hydrogen or halogen, and
R37 may be hydrogen or halogen.

For example, said PDE4-selective inhibitor or the present application may comprise Roflumilast or a derivative thereof.

For example, said derivative of Roflumilast may comprise benzamide, bezafibrate, and/or 4-Methylbenzanilide.

For example, said compound having the structure shown in Formula I may comprise a compound set forth in the patent application (US5712298A), which is incorporated herein by reference.

For example, in Formula I: R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine,
R2 may be C₁-C₄ alkoxy fully or partially substituted with fluorine, and
R3 may be phenyl, pyridinyl, phenyl substituted with R31, R32, and R33, or pyridinyl substituted with R34, R35, R36, and R37, wherein:
   R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, in Formula I: R1 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, R2 may be methoxy fully or partially substituted with fluorine, and R3 may be phenyl, pyridinyl, phenyl substituted with R31, R32 and R33, or pyridinyl substituted with R34, R35, R36 and R37, wherein R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, in Formula I: R1 may be C₃-C₇-cycloalkoxy, C₃-C₇-cycloalkylmethoxy or benzyloxy, R2 may be C₁-C₄ alkoxy fully or partially substituted with fluorine, and R3 may be phenyl, pyridinyl, phenyl substituted with R31, R32, and R33, or pyridinyl substituted with R34, R35, R36, and R37, wherein:
R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, in Formula I: R1 may be C₁-C₄-alkoxy fully or partially substituted with fluorine, R2 may be hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and R3 may be phenyl, pyridinyl, phenyl substituted with R31, R32, and R33, or pyridinyl substituted with R34, R35, R36, and R37, wherein:
R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, in Formula I: R1 may be C₁-C₄-alkoxy fully or partially substituted with fluorine, R2 may be C₃-C₇-cycloalkylmethoxy or benzyloxy, and R3 may be phenyl, pyridinyl, phenyl substituted with R31, R32, and R33, or pyridinyl substituted with R34, R35, R36, and R37, wherein:
R31 may be hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 may be hydrogen, hydroxyl, halogen, amino, trifluoromethyl, C₁-C₄-alkyl or C₁-C₄-alkoxy, R33 may be hydrogen, halogen, C₁-C₄-alkyl or C₁-C₄-alkoxy, R34 may be hydroxyl, halogen, cyano, carboxyl, C₁-C₄-alkyl, C₁-C₄-alkoxy, C₁-C₄-alkoxycarbonyl or amino, R35 may be hydrogen, halogen, amino or C₁-C₄-alkyl, R36 may be hydrogen or halogen, and R37 may be hydrogen or halogen.

For example, in Formula I: R1 may be C₁-C₄-alkoxy, Cs-Cs-cycloalkoxy, C₃-C₅-cycloalkylmethoxy, benzyloxy, or C₁-C₄-alkoxy fully or partially substituted with fluorine, R2 may be C₁-C₄-alkoxy fully or partially substituted with fluorine, and R3 may be phenyl, pyridinyl, phenyl substituted with R31, R32, and R33, or pyridinyl substituted with R34, R35, R36, and R37, wherein:
R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, in Formula I: R1 may be C₁-C₄-alkoxy, Cs-Cs-cycloalkoxy, C₃-C₅-cycloalkylmethoxy, benzyloxy, or C₁-C₄-alkoxy fully or partially substituted with fluorine, R2 may be methoxy fully or partially substituted with fluorine, and R3 may be phenyl, pyridinyl, phenyl substituted with R31, R32, and R33, or pyridinyl substituted with R34, R35, R36, and R37, wherein:
R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, in Formula I: R1 may be Cs-Cs-cycloalkoxy, Cs-Cs-cycloalkylmethoxy, or benzyloxy, R2 may be C₁-C₄-alkoxy fully or partially substituted with fluorine, and R3 may be phenyl, pyridinyl, phenyl substituted with R31, R32, and R33, or pyridinyl substituted with R34, R35, R36, and R37, wherein:
R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, in Formula I: R1 may be C₁-C₄-alkoxy fully or partially substituted with fluorine, R2 may be C₁-C₄-alkoxy, Cs-Cs-cycloalkoxy, Cs-Cs-cycloalkylmethoxy, benzyloxy, or C₁-C₄-alkoxy fully or partially substituted with fluorine, and R3 may be phenyl, pyridinyl, phenyl substituted with R31, R32, and R33, or pyridinyl substituted with R34, R35, R36, and R37, wherein:
R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, in Formula I: R1 may be C₁-C₄-alkoxy fully or partially substituted with fluorine, R2 may be Cs-Cs-cycloalkylmethoxy or benzyloxy, and R3 may be phenyl, pyridinyl, phenyl substituted with R31, R32, and R33, or pyridinyl substituted with R34, R35, R36, and R37, wherein:
R31 may be halogen, cyano, carboxyl, C₁-C₄-alkyl, C₁-C₄-alkoxy or C₁-C₄-alkoxycarbonyl, R32 may be hydrogen, halogen, C₁-C₄-alkyl or C₁-C₄-alkoxy, R33 may be hydrogen, halogen, C₁-C₄-alkyl or C₁-C₄-alkoxy, R34 may be halogen or C₁-C₄-alkyl, R35 may be hydrogen or halogen, R36 may be hydrogen or halogen, and R37 may be hydrogen or halogen.

For example, in Formula I: R1 may be C₁-C₄-alkoxy, C₁-C₄-cycloalkoxy, C₁-C₄-cycloalkylmethoxy, benzyloxy, or C₁-C₄-alkoxy fully or partially substituted with fluorine, R2 may be C₁-C₄-alkoxy fully or partially substituted with fluorine, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be C₁-C₄-alkoxy, Cs-Cs-cycloalkoxy, C₃-C₅-cycloalkylmethoxy, benzyloxy, or C₁-C₄-alkoxy fully or partially substituted with fluorine, R2 may be methoxy fully or partially substituted with fluorine, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be Cs-Cs-cycloalkoxy, Cs-Cs-cycloalkylmethoxy or benzyloxy, R2 may be C₁-C₄-alkoxy fully or partially substituted with fluorine, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be C₁-C₄-alkoxy fully or partially substituted with fluorine, R2 may be C₁-C₄-alkoxy, Cs-Cs-cycloalkoxy, Cs-Cs-cycloalkylmethoxy, benzyloxy or C₁-C₄-alkoxy fully or partially substituted with fluorine, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be C₁-C₄-alkoxy fully or partially substituted with fluorine, R2 may be Cs-Cs-cycloalkylmethoxy or benzyloxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be difluoromethoxy, R2 may be methoxy, ethoxy, isopropoxy, isobutoxy, cyclopentoxy, cyclopropylmethoxy, cyclobutylmethoxy, difluoromethoxy or 2,2,2-trifluoroethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

For example, in Formula I: R1 may be difluoromethoxy, R2 may be methoxy, cyclopropylmethoxy, cyclobutylmethoxy or difluoromethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be methoxy, n-propoxy, n-butoxy, cyclopropylmethoxy or 2,2,2-trifluoroethoxy, R2 may be difluoromethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, in Formula I: R1 may be difluoromethoxy, R2 may be cyclopropylmethoxy, and R3 may be 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

For example, the compound having the structure of Formula I may comprise N-(3,5-dichloropyridin-4-yl)-4-difluoromethoxy-3-methoxybenzamide (melting point: 170°C), N-(3,5-dichloropyridin-4-yl)-3,4-bis(difluoromethoxy)benzamide (melting point: 134°C), N-(3,5-dichloropyridin-4-yl)-3-cyclobutylmethoxy-4-difluoromethoxybenzamide (melting point: 155°C), N-(3,5-dichloropyridin-4-yl)-3-cyclopentoxy-4-difluoromethoxybenzamide (melting point: 128.5-129°C), N-(3,5-dichloropyridin-4-yl)-3-cyclopropylmethoxy-4-difluoromethoxybenzamide (melting point: 158°C), N-(3,5-dichloro-2,6-difluoropyridin-4-yl)-3-difluoromethoxy-4-methoxybenzamide (melting point: 218°C)), N-(2,6-dichlorophenyl)-3-difluoromethoxy-4-methoxybenzamide (melting point: 164°C), N-(2,6-dimethylphenyl)-3-difluoromethoxy-4-methoxybenzamide (melting point: 164°C), N-(2-chloropyridin-3-yl)-3-difluoromethoxy-4-methoxybenzamide (melting point: 165°C), N-(3,5-dibromopyridin-2-yl)-3-difluoromethoxy-4-methoxybenzamide (melting point: 143°C), N-(3,5-dichloropyridin-4-yl)-3-difluoromethoxy-4-methoxybenzamide (melting point: 178°C), N-(3,5-dichloropyridin-4-yl)-3-difluoromethoxy-4-propoxybenzamide (melting point: 159°C), N-(3,5-dichloropyridin-4-yl)-3-ethoxy-4-difluoromethoxybenzamide (melting point: 134°C), N-(3,5-dichloropyridin-4-yl)benzyloxy-3-difluoromethoxybenzamide (melting point: 152°C), N-(3,5-dichloropyridin-4-yl)-4-butoxy-3-difluoromethoxybenzamide (melting point: 146°C), N-(3,5-dichloropyridin-4-yl)-4-cyclopropylmethoxy-3-difluoromethoxybenzamide (melting point: 159°C), N-(3,5-dichloropyridin-4-yl)-4-difluoromethoxy-3-(1-methylethoxy)benzamide (melting point: 99.5°C), N-(3,5-dichloropyridin-4-yl)-4-difluoromethoxy-3-(2,2,2-trifluoroethoxy)-benzamide (melting point: 147°C), N-(3,5-dichloropyridin-4-yl)-4-difluoromethoxy-3-(2-methylpropoxy)benzamide (melting point: 153°C), N-(2,3,5,6-tetrafluoropyridin-4-yl)-4-difluoromethoxy-3-methoxybenzamide (melting point: 146°C), N-(2,4,6-trifluorophenyl)-4-difluoromethoxy-3-methoxybenzamide (melting point: 145°C), N-(2,6-dichloro-4-ethoxycarbonylphenyl)-4-difluoromethoxy-3-methoxybenzamide (melting point: 176°C), N-(2,6-dichlorophenyl)-4-difluoromethoxy-3-methoxybenzamide (melting point: 186°C), N-(2,6-difluorophenyl)-4-difluoromethoxy-3-methoxybenzamide (melting point: 139°C), N-(2,6-dimethylphenyl)-4-difluoromethoxy-3-methoxybenzamide (melting point: 143°C), N-(2-bromophenyl)-4-difluoromethoxy-3-methoxybenzamide (melting point: 121°C), N-(2-chloro-6-methylphenyl)-4-difluoromethoxy-3-methoxybenzamide (melting point: 144°C), N-(2-chloropyridin-3-yl)-4-difluoromethoxy-3-methoxybenzamide (melting point: 137.5°C), N-(2-methylphenyl)-4-difluoromethoxy-3-methoxybenzamide (melting point: 125°C), N-(3,5-dibromopyridin-2-yl)-4-difluoromethoxy-3-methoxybenzamide (melting point: 141°C), N-(3,5-dichloro-2,6-difluoropyridin-4-yl)-4-difluoromethoxy-3-methoxybenzamide (melting point: 174°C), N-(3-chloro-2,5,6-trifluoropyridin-4-yl)-4-difluoromethoxy-3-methylbenzamide (melting point: 141°C), and N-(3-methylpyridin-2-yl)-4-difluoromethoxy-3- methoxybenzamide (melting point: 96°C).

For example, suitable salts of the compound of Formula I, depending on the substitution reaction, may be all acid addition salts or all salts formed with bases. Particular mention should be made of salts prepared from pharmacologically tolerable inorganic and organic acids and bases that can usually be used pharmaceutically. Pharmacologically intolerable salts, such as in preparation of the compounds described herein, may first be precipitated as intermediate products on an industrial scale and then converted into pharmacologically tolerable salts by means familiar to person skilled in the art. In one aspect, the suitable salts may be water-soluble and water-insoluble acid addition salts formed with acids, such as acids suitable for this purpose, wherein, in preparing the salts, the acids used may be monobasic or polybasic acids as desired, and the feed ratio may be quantified in moles or diverted from the quantitative ratio according to what kind of salt is needed.

For example, the salts can also be salts formed with alkali. Examples of alkaline salts are mentioned as suitable salts, where the alkali can be quantified in moles or diverted from the quantitative ratio when preparing the salts.

### Immune Checkpoint Inhibitor

In one aspect, the immune checkpoint inhibitor of the present application may comprise an antagonist of one or more proteins of CTLA-4, PD-1, PD-L1, PD-L2, LAG-3, TIM-3, galectin-9, CEACAM-1, BTLA, CD69, galectin-1, TIGIT, CD113, GPR56, VISTA, 2B4, CD48, GARP, PD1H, LAIR1, TIM-1, and TIM-4.

For example, the immune checkpoint inhibitor of the present application may comprise an antagonist of PD-1 and/or PD-L1. For example, the immune checkpoint inhibitor of the present application may be an antagonist of PD-1 and/or PD-L1.

For example, an antagonist of PD-1 and/or PD-L1 of the present application may inhibit the interaction between PD-1 and PD-L1.

For example, an antagonist of PD-1 and/or PD-L1 of the present application may comprise an antibody specifically binding to PD-1 and/or PD-L1 or an antigen binding fragment thereof.

For example, the antibody specifically binding to PD-1 and/or PD-L1 or antigen binding fragment thereof may comprise a HCDR3 comprising an amino acid sequence set forth in any one of SEQ ID NOs: 3, 13, 23, and 33. For example, the antibody specifically binding to PD-1 and/or PD-L1 or antigen binding fragment thereof may comprise a HCDR2 comprising an amino acid sequence set forth in any one of SEQ ID NOs: 2, 12, 22, and 32. For example, the antibody specifically binding to PD-1 and/or PD-L1 or antigen binding fragment thereof may comprise a HCDR1 comprising an amino acid sequence set forth in any one of SEQ ID NOs: 1, 11, 21, and 31. For example, the CDRs of the antibody of the present application may be divided according to the Kabat scheme.

For example, the antibody specifically binding to PD-1 and/or PD-L1 or antigen binding fragment thereof may comprise a HCDR3 comprising an amino acid sequence set forth in any one of SEQ ID NOs: 3, 13, 23, and 33, the antibody specifically binding to PD-1 and/or PD-L1 or antigen binding fragment thereof may comprise a HCDR2 comprising an amino acid sequence set forth in any one of SEQ ID NOs: 2, 12, 22, and 32, and the antibody specifically binding to PD-1 and/or PD-L1 or antigen binding fragment thereof may comprise a HCDR1 comprising an amino acid sequence set forth in any one of SEQ ID NOs: 1, 11, 21, and 31. For example, the CDRs of the antibody of the present application may be divided according to the Kabat scheme.

For example, the antibody specifically binding to PD-1 and/or PD-L1 or antigen binding fragment thereof may comprise an LCDR1 comprising an amino acid sequence set forth in any one of SEQ ID NOs: 6, 16, 26, and 36. For example, the antibody specifically binding to PD-1 and/or PD-L1 or antigen binding fragment thereof may comprise an LCDR2 comprising an amino acid sequence set forth in any one of SEQ ID NOs: 5, 15, 25, and 35. For example, the antibody specifically binding to PD-1 and/or PD-L1 or antigen binding fragment thereof may comprise an LCDR1 comprising an amino acid sequence set forth in any one of SEQ ID NOs: 4, 14, 24, and 34. For example, the CDRs of the antibody of the present application may be divided according to the Kabat scheme.

For example, the antibody specifically binding to PD-1 and/or PD-L1 or antigen binding fragment thereof may comprise an LCDR3 comprising an amino acid sequence set forth in any one of SEQ ID NOs: 6, 16, 26, and 36, the antibody specifically binding to PD-1 and/or PD-L1 or antigen binding fragment thereof may comprise an LCDR2 comprising an amino acid sequence set forth in any one of SEQ ID NOs: 5, 15, 25, and 35, and the antibody specifically binding to PD-1 and/or PD-L1 or antigen binding fragment thereof may comprise an LCDR1 comprising an amino acid sequence set forth in any one of SEQ ID NOs: 4, 14, 24, and 34. For example, the CDRs of the antibody of the present application may be divided according to the Kabat scheme.

For example, the antibody specifically binding to PD-1 and/or PD-L1 or antigen binding fragment thereof may comprise a HCDR3 comprising an amino acid sequence set forth in any one of SEQ ID NOs: 3, 13, 23, and 33, and the antibody specifically binding to PD-1 and/or PD-L1 or antigen binding fragment thereof may comprise an LCDR3 comprising an amino acid sequence set forth in any one of SEQ ID NOs: 6, 16, 26, and 36. For example, the CDRs of the antibody of the present application may be divided according to the Kabat scheme.

For example, the antibody specifically binding to PD-1 and/or PD-L1 or antigen binding fragment thereof may comprise a HCDR3 comprising an amino acid sequence set forth in any one of SEQ ID NOs: 3, 13, 23, and 33, the antibody specifically binding to PD-1 and/or PD-L1 or antigen binding fragment thereof may comprise a HCDR2 comprising an amino acid sequence set forth in any one of SEQ ID NOs: 2, 12, 22, and 32, the antibody specifically binding to PD-1 and/or PD-L1 or antigen binding fragment thereof may comprise a HCDR1 comprising an amino acid sequence set forth in any one of SEQ ID NOs: 1, 11, 21, and 31, the antibody specifically binding to PD-1 and/or PD-L1 or antigen binding fragment thereof may comprise an LCDR3 comprising an amino acid sequence set forth in any one of SEQ ID NOs: 6, 16, 26, and 36, the antibody specifically binding to PD-1 and/or PD-L1 or antigen binding fragment thereof may comprise an LCDR2 comprising an amino acid sequence set forth in any one of SEQ ID NOs: 5, 15, 25, and 35, and the antibody specifically binding to PD-1 and/or PD-L1 or antigen binding fragment thereof may comprise an LCDR1 comprising an amino acid sequence set forth in any one of SEQ ID NOs: 4, 14, 24, and 34. For example, the CDRs of the antibody of the present application may be divided according to the Kabat scheme.

For example, the antibody specifically binding to PD-1 and/or PD-L1 or antigen binding fragment thereof may comprise a VH comprising an amino acid sequence set forth in any one of SEQ ID NOs: 7, 17, 27, and 37. For example, the antibody specifically binding to PD-1 and/or PD-L1 or antigen binding fragment thereof may comprise a VL comprising an amino acid sequence set forth in any one of SEQ ID NOs: 4, 14, 24, and 34. For example, the CDRs of the antibody of the present application may be divided according to the Kabat scheme.

For example, the antibody specifically binding to PD-1 and/or PD-L1 or antigen binding fragment thereof may comprise a VH comprising an amino acid sequence set forth in any one of SEQ ID NOs: 7, 17, 27, and 37, and the antibody specifically binding to PD-1 and/or PD-L1 or antigen binding fragment thereof may comprise a VL comprising an amino acid sequence set forth in any one of SEQ ID NOs: 4, 14, 24, and 34.

For example, the antibody specifically binding to PD-1 and/or PD-L1 or antigen binding fragment thereof may comprise a heavy chain comprising an amino acid sequence set forth in any one of SEQ ID NOs: 9, 19, 29, and 39. For example, the antibody specifically binding to PD-1 and/or PD-L1 or antigen binding fragment thereof may comprise a light chain comprising an amino acid sequence set forth in any one of SEQ ID NOs: 10, 20, 30, and 40.

For example, the antibody specifically binding to PD-1 and/or PD-L1 or antigen binding fragment thereof may comprise a heavy chain comprising an amino acid sequence set forth in any one of SEQ ID NOs: 9, 19, 29, and 39, and the antibody specifically binding to PD-1 and/or PD-L1 or antigen binding fragment thereof may comprise a light chain comprising an amino acid sequence set forth in any one of SEQ ID NOs: 10, 20, 30, and 40.

For example, the antibody may be selected from the group consisting of a chimeric antibody, a humanized antibody, and a fully human antibody.

For example, the antigen binding fragment may be selected from the group consisting of Fab, Fv, scFv, Fab ', and (Fab') 2.

For example, the antibody specifically binding to PD-1 and/or PD-L1 of the present application may comprise:
HCDR1-3 and LCDR1-3 of nivolumab (Opdivo or Nivolumab),
HCDR1-3 and LCDR1-3 of pembrolizumab (Keytruda or Pembrolizumab),
HCDR1-3 and LCDR1-3 of Toripalimab (JS-001, CAS #: 1924598-82-2),
HCDR1-3 and LCDR1-3 of Sintilimab (IBI308, CAS #: 2072873-06-2),
HCDR1-3 and LCDR1-3 of Camrelizumab (SHR-1210, CAS #: 1798286-48-2),
HCDR1-3 and LCDR1-3 of BMS-936559 (MDX-1105);
HCDR1-3 and LCDR1-3 of MPDL3280A (Tecentriq or Atezolizumab);
HCDR1-3 and LCDR1-3 ofMEDI4736 (Imfinzi or Durvalumab); or
HCDR1-3 and LCDR1-3 of MSB0010718C (Avelumab); for example, the CDRs of the antibody of the present application may be divided according to the Kabat scheme; for example, the CDRs of the antibody of the present application may be divided according to the Chothia scheme; for example, the CDRs of the antibody of the present application may be divided according to the IMGT scheme.

For example, the antibody specifically binding to PD-1 and/or PD-L1 of the present application may comprise:
a VH and a VL of nivolumab,
a VH and a VL of pembrolizumab,
a VH and a VL of BMS-936559;
a VH and a VL of MPDL3280A;
a VH and a VL of MEDI4736; or
a VH and a VL of MSB0010718C.

For example, the antibody specifically binding to PD-1 and/or PD-L1 of the present application can be selected from the group consisting of nivolumab, pembrolizumab, and BMS-936559 (also known as 12A4 or MDX-1105, the description of which can be found in U.S. Patent No. 7,943,743 or WO2013/173223A1), MPDL3280A (also known as RG7446 or Atezolizumab, the description of which can be found in Herbst et al. (2013) J Clin Oncol 31 (suppl): 3000. Abstract., or U.S. Patent No. 8,217,149), MEDI4736 (also known as Durvalumab, the description of which can be found in Khleif (2013) In: Proceedings from the European Cancer Congress 2013; September 27 to October 1, 2013; Amsterdam, The Netherlands), AMP-224 (also known as B7-DC Fc fusion protein, the description of which can be found in U.S. Publication No. US2013/0017199A1) and MSB0010718C (also known as Avelumab, the description of which can be found in US 2014/0341917A1).

### Carriers and Formulations

The PDE4-selective inhibitor of the present application and the immune checkpoint inhibitor of the present application can each be separate formulations (i.e., the first formulation, the second formulation, and the third formulation), or can be prepared into a suitable formulation in the form of composition, e.g., the PDE4-selective inhibitor of the present application and the immune checkpoint inhibitor of the present application. Both the separate formulations and the formulation prepared in the form of composition can be coordinated with any suitable carrier to obtain a desired formulation form.

For example, the dosage form of the first formulation, the second formulation, or the pharmaceutical composition can comprise tablets, capsules, granules, powders, syrups, suspensions, suppositories, ointments, creams, gels, patches, inhalants, injections, and the like. These formulations can be prepared according to conventional methods.

For example, in the case of liquid formulation, it can be in a form which is dissolved or suspended in water or another suitable solvent when used.

For example, tablets and granules can be coated by a well-known method.

For example, in the case of injection, it can be prepared by dissolving the active ingredient (comprising the PDE4-selective inhibitor and/or the immune checkpoint inhibitor of the present application) into water, or into normal saline or glucose solution as needed. In addition, buffer or preservative can be added, for example.

For example, it can also be provided in any form of formulation for oral administration or non-oral administration. For example, it can be prepared into an oral pharmaceutical formulation in form of granules, fine granules, powders, hard capsules, soft capsules, syrups, emulsions, suspensions, liquids, or the like.

For example, it can also be a non-oral pharmaceutical formulation in form of injections, infusions, transdermal delivery formulations, transmucosal delivery formulation, nose drops, inhalants, suppositories or the like for intravenous, intramuscular, intradermal, intracavitary, intratumoral, peritumoral or subcutaneous administration.

For example, injections, infusions, or the like can be prepared into powdered dosage forms such as lyophilized forms, which can be reconstituted in a suitable aqueous media such as normal saline for use.

For example, a sustained-release formulation coated by polymers or the like can also be administered directly to organs and tissues such as brain, blood, lymph, or the like, or into umors. For example, the sustained-release formulation can comprise a sustained-release pump, a sustained-release capsule, a sustained-release agent, an implant, or a sustained-release implant.

The types of additives (carriers), the ratio of the additives (carriers) to the active ingredients, or the manufacturing method of the formulation used in the manufacture of the pharmaceutical formulation can be appropriately selected by a person skilled in the art according to the form of the formulation. For example, inorganic or organic substances, or solid or liquid substances can be used as additives (carriers) for formulation, e.g., in amount between 1 wt % and 90 wt % based on the weight of the active ingredient.

For example, in the manufacture of solid formulations for oral administration, the active ingredients (comprising the PDE4-selective inhibitor and/or the immune checkpoint inhibitor of the present application) can be, e.g., mixed with carrier ingredients to produce powders; for example, granules can be added as needed; for example, in manufacture of tablets, the powders and/or granules can be directly pressed into tablets; for example, they can be further incorporated with lubricants for pressing tablets; for example, the granules or tablets can also be coated with an enteric matrix such as a polymer to prepare an enteric formulation; for example, they can be prepared into a long-acting formulation by coating; for example, in the manufacture of capsule, the powders and/or granules can be filled into a hard capsule; for example, the active ingredients can also be prepared into a soft capsule by coating with a gelatin film directly or after dissolved in a solvent.

For example, in the manufacture of injection, the active ingredients can be dissolved in distilled water for injection, together with desired substances as needed; for example, they can further be subject to sterile filtration and then filled in ampoules; for example, they can be further incorporated with desired substances and lyophilized in vacuum to produce a ready-to-use injection. For example, the active ingredients can also be incorporated with desired substances and emulsified in water to produce an emulsion for injection.

For example, in the manufacture of agent for rectal administration, the active ingredients can be humidified and dissolved, together with suppository base, and then flowed into a mold for cooling, e.g., the active ingredients can also be dissolved in a desired substance and coated with a gelatin film.

For example, in the manufacture of topical agent for skin, the active ingredients can be added to a desired substance, humidified as need, and kneaded to produce an ointment; for example, they can also be kneaded with a binder such as a desired substance, and then spreaded onto a non-woven fabric such as polyalkyls to produce a strip formulation.

For example, they can also be prepared into a sustained-release formulation such as an implant or a delivery system encapsulated in a microcapsule, which can be prepared using a carrier capable of preventing immediate clearance from the body. For example, desired substances can be used. Those skilled in the art can readily prepare these materials. For example, a liposome suspension can also be used as a pharmaceutically acceptable carrier. The liposome can be prepared to comprise the desired substances, which can be prepared by making them to a suitable size for use using a filter with appropriate pore size, and purified by reverse phase evaporation.

In the present application, the dosage and frequency of administrations can be appropriately selected according to conditions such as prevention and/or treatment purpose of disease progression in a treatment object (subject), the type of disease, weight, and age of the patient, and the like. For example, in the case of oral administration, it can be divided into suitable doses, or administered every few days. In the case of injection, it can be administered continuously or intermittently.

Without wishing to be limited by any theory, the examples hereinafter are merely intended to illustrate the pharmaceutical combinations, methods of use, and uses of the present application, and are not intended to limit the scope of the present invention.

### EXAMPLES

### Example 1. Roflumilast Capable Of Inhibiting Tumor Metastasis

C57 mice used in the present application were purchased from Vital River Laboratory Animal Technology Co., Ltd.; MC38 cells were purchased from ATCC; Roflumilast was purchased from Aladdin under article number of R126602; and Oxaliplatin was purchased from Aladdin under article number of 0124003. In use, DMSO was used as the solvent for Oxaliplatin and Roflumilast.
(1) Tumorigenesis in mice. 6-8 week-old C57 female mice were selected. MC38 cells were cultured *in vitro* to logarithmic phase, collected, and resuspended in a PBS buffer solution. The collected cells were injected into the spleen of mice through surgery with 5 × 10⁵ MC38 cells per mouse to establish a tumor metastasis model.
(2) After modeling, the mice were divided into three experimental groups including a blank control, a Roflumilast group, and an oxaliplatin group. The mice in the Roflumilast group were sequentially intraperitoneally injected with 4 mg/kg of Roflumilast every 3 days, with 6 doses in total; while the oxaliplatin group was injected with 4 mg/kg of oxaliplatin, with 6 doses in total; and the blank control was injected with the same dose of normal saline. The day of the first injection was taken as Day 1. Then, the mice were detected for weight and recorded, and subject to Luciferin fluorescence imaging. That is, each mouse was intraperitoneally injected with 100 µL of 1 mg/mL aqueous solution of potassium D-Luciferin as formulated. 10 min after injection, the mice were subject to Luciferin fluorescence imaging using a Small Animal Liver Imaging System (model: IVIS Lumina XRMS Series III).

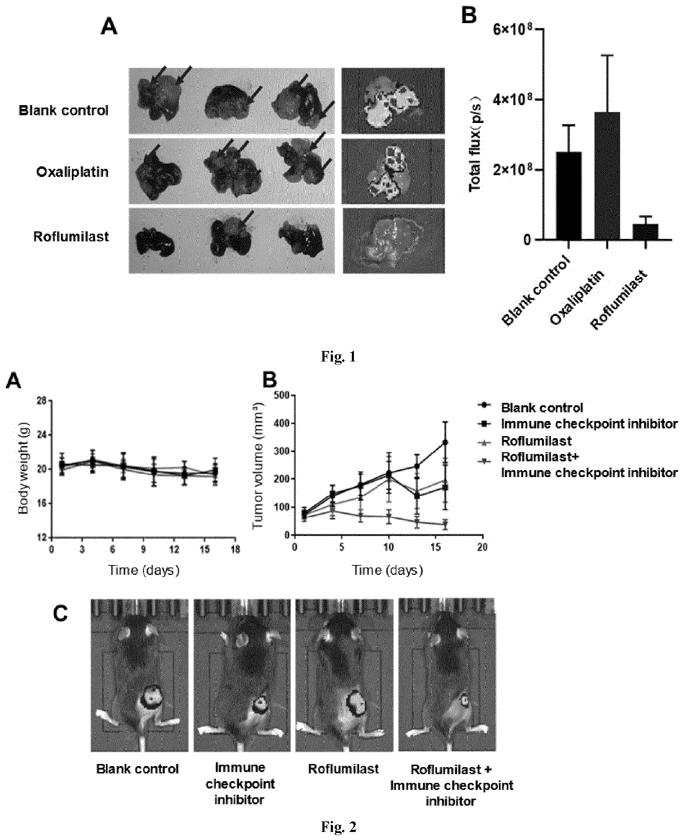

As shown in the results of FIGs. 1A and 1B, the PDE4-selective inhibitor of the present application can inhibit the tumor metastasis and/or have good safety.

### Example 2. PDE4-Selective Inhibitor Capable Of Synergistically Inhibiting Tumor Growth With Immune Checkpoint Inhibitor

(1) Tumorigenesis in mice. 6-8 week-old C57 female mice were selected. MC38 cells were cultured *in vitro* to logarithmic phase, collected, and resuspended in a PBS buffer solution. The collected cells were subcutaneously injected into the leg of mice with 5 × 10⁵ MC38 cells per mouse to establish a subcutaneous tumor model.
(2) After tumorigenesis, the mice were divided into four experimental groups including a blank control, a Roflumilast group, an immune checkpoint inhibitor group, and a Roflumilast + immune checkpoint inhibitor group. The mice in the Roflumilast + immune checkpoint inhibitor group were sequentially intraperitoneally injected with 4 mg/kg of Roflumilast and 2 mg/kg of immune checkpoint inhibitor (e.g., a PD-L1 antibody, such as, MPDL3280A, MEDI4736 or MSB0010718C) every 3 days, with 6 doses in total, while the Roflumilast group and the immune checkpoint inhibitor group were separately injected with the same dose of Roflumilast and the immune checkpoint inhibitor, and the blank control was injected with the same dose of normal saline. The day of the first injection was taken as day 1, the mice were detected for weight and tumor size and recorded: the tumor volume (mm³) = length (L) × width (W)²/2, and then subject to Luciferin luorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

As shown in the results of FIGs. 2A, 2B, and 2C, the PDE4-selective inhibitor of the present application in combination with the immune checkpoint inhibitor can inhibit the tumor growth, which have synergistic effects and/or have good safety.

### 2.1 Roflumilast Synergistically Inhibiting Growth Of Colon Cancer With Anti-PD-L1 Antibody.

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mice to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with, e.g., 4 mg/kg of PDE4 inhibitor and, e.g., 2 mg/kg of immune checkpoint inhibitor (e.g., a PD-L1 antibody) every 3 days, with 6 doses in total, while the PDE4 inhibitor group and the immune checkpoint inhibitor group were separately injected with the same dose of PDE4 inhibitor and the immune checkpoint inhibitor in the same manner, and the blank control was injected with the same dose of normal saline. The day of the first injection was taken as day 1. The mice were detected for the weight and tumor size and recorded: the tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Liver Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.2 Roflumilast Synergistically Inhibiting Growth Of Breast Cancer With Anti-PD-L1 Antibody

(1) Tumorigenesis in mice. 6-8 week-old C57 female mice were selected. Breast cancer cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.3 Roflumilast Synergistically Inhibiting Growth Of Melanoma With Anti-PD-L1 Antibody

(1) Tumorigenesis in mice. 6-8 week-old C57 female mice were selected. Melanoma cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.4 Roflumilast Synergistically Inhibiting Growth Of Lymphoma With Anti-PD-L1 Antibody.

(1) Tumorigenesis in mice. 6-8 week-old C57 female mice were selected. Lymphoma cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.5 Roflumilast Synergistically Inhibiting Growth Of Hematologic Tumor With Anti-PD-L1 Antibody

(1) Tumorigenesis in mice. 6-8 week-old C57 female mice were selected. Hematologic tumor cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.6 Roflumilast Synergistically Inhibiting Growth Of Colon Cancer With Anti-PD-1 Antibody

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody, such as Nivolumab, Pembrolizumab, Toripalimab, Sintilimab, Camrelizumab and the like) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.7 Roflumilast Synergistically Inhibiting Growth Of Breast Cancer With Anti-PD-1 Antibody

(1) Tumorigenesis in mice. 6-8 week-old C57 female mice were selected. Breast cancer cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.8 Roflumilast Synergistically Inhibiting Growth Of Melanoma With Anti-PD-1 Antibody

(1) Tumorigenesis in mice. 6-8 week-old C57 female mice were selected. Melanoma cells were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.9 Roflumilast Synergistically Inhibiting Growth Of Colon Cancer With Anti-CTLA-4 Antibody

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., CTLA-4 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.10 Roflumilast Synergistically Inhibiting Growth Of Colon Cancer With Anti-LAG-3 Antibody

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor and e.g. 2 mg/kg of immune checkpoint inhibitor (e.g., LAG-3 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.11 Roflumilast Derivative Synergistically Inhibiting Growth Of Colon Cancer With Anti-PD-L1 Antibody

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4 inhibitor (e.g., Roflumilast derivatives) and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subj ect to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### 2.12 Another PDE4-Selective Inhibitor Synergistically Inhibiting Growth Of Colon Cancer With Anti-PD-L1 Antibody

(1) Tumorigenesis in mice. 6-8 week-old C57 female mice were selected. Colon cancer cells, such as MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, the mice were divided into four experimental groups including a blank control, a PDE4 inhibitor group, an immune checkpoint inhibitor group, and a PDE4 inhibitor + immune checkpoint inhibitor group. The mice in the PDE4 inhibitor + immune checkpoint inhibitor group were sequentially intraperitoneally injected with e.g. 4 mg/kg of PDE4-selective inhibitor (e.g., Apremilast, Crisaborole, Drotaverine, CC-1088, BPN14770, GSK356278, HT-0712, TAK-648, Zembrin, ASP9831, Etazolate, Piclamilast, Rolipram, Cilomilast, GSK256066, AWD 12-281, Quinolyl oxazole, DC-TA-46, Filaminast and/or Glaucine) and e.g. 2 mg/kg of immune checkpoint inhibitor (e.g., an PD-L1 antibody) every 3 days, with 6 doses in total, while the PDE4 inhibitor group and the immune checkpoint inhibitor group were separately injected with the same dose of PDE4 inhibitor and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that the PDE4-selective inhibitor of the present application in combination with the immune checkpoint inhibitor can inhibit the tumor growth, which have synergistic effects and/or have good safety.

### Example 3 Selective Inhibitors Other Than PDE4-Selective Inhibitor Producing No Significant Synergistic Anti-Tumor Effect In Combination With Immune Checkpoint Inhibitor

(1) Tumorigenesis in mice. 6-8 week-old C57 female mice were selected. Tumor cells, such as colon cancer cells, e.g., MC38 cells, were cultured *in vitro* to logarithmic phase, collected, and resuspended in a PBS buffer solution. The collected cells were subcutaneously injected into the leg of mice with 5 × 10⁵ cells per mouse to establish a subcutaneous tumor model.
(2) After tumorigenesis, mice were divided into four experimental groups: blank control, other PDE inhibitors group (e.g., PDE5-selective inhibitor such as Tadalafil, Sildenafil citrate, Kuraridine, and the like), immune checkpoint inhibitor group, and other PDE inhibitors + immune checkpoint inhibitor group. Mice in the other PDE inhibitors + immune checkpoint inhibitor group were sequentially intraperitoneally injected with e.g. 4 mg/kg of other PDE-selective inhibitors and e.g. 2 mg/kg of immune checkpoint inhibitor (e.g., an PD-L1 antibody) every 3 days, with 6 doses in total, the other PDE inhibitors group and the immune checkpoint inhibitor group were separately injected with the same dose of other PDE inhibitors and immune checkpoint inhibitor in the same manner, and the blank control was injected with the same dose of normal saline. The day of the first injection was taken as day 1. The mice were detected for weight and tumor size and recorded: tumor volume (mm³) = length (L) × width (W)²/2, and then subject to Luciferin fluorescence imaging of tumor. That is, each mouse was intraperitoneally injected with 100 µL of 1 mg/mL aqueous solution of D-Luciferin potassium as formulated. 10 min after injection, the mice were subject to Luciferin fluorescence imaging using a Small Animal Live Imaging System (model: IVIS Lumina XRMS Series III).

The results show that selective inhibitors other than PDE4-selective inhibitor, such as PDE5-selective inhibitor, have no significant synergistic anti-tumor effect in combination with immune checkpoint inhibitor.

### Example 4 PDE4 Inhibitor Inhibiting Metastasis

Different concentrations of PDE4 inhibitors, such as 5 µM, 10 µM, or 20 µM, were administered to verify the inhibitory effect on metastasis in the scratch test of MC38. The PDE4 inhibitors can be Roflumilast, BPN14770 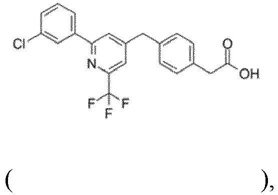 GSK256066 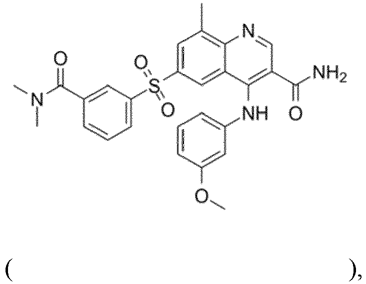 Rolipram 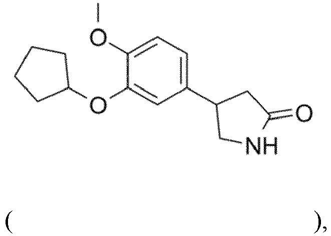 or Dipyridamole

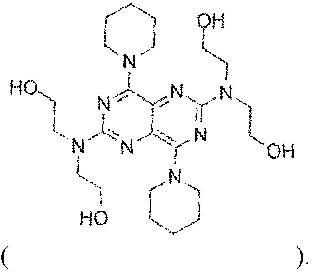

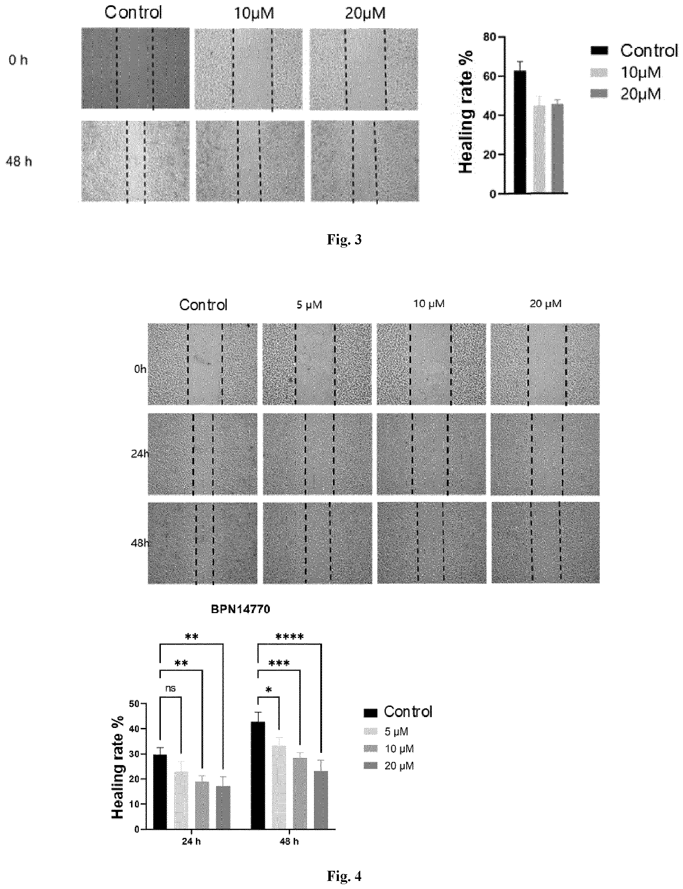

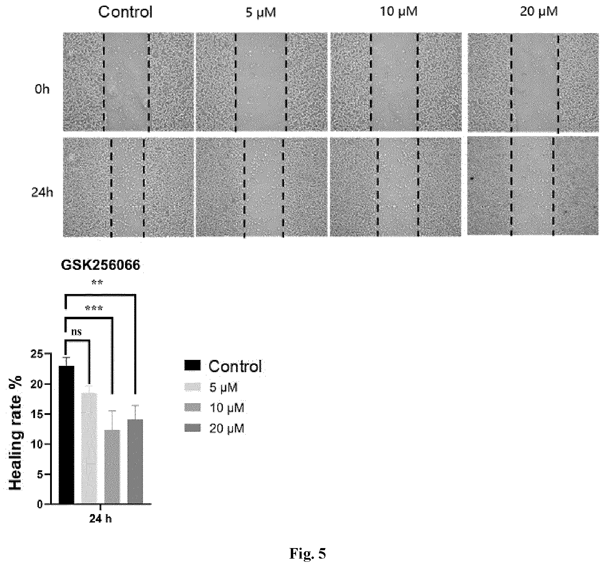

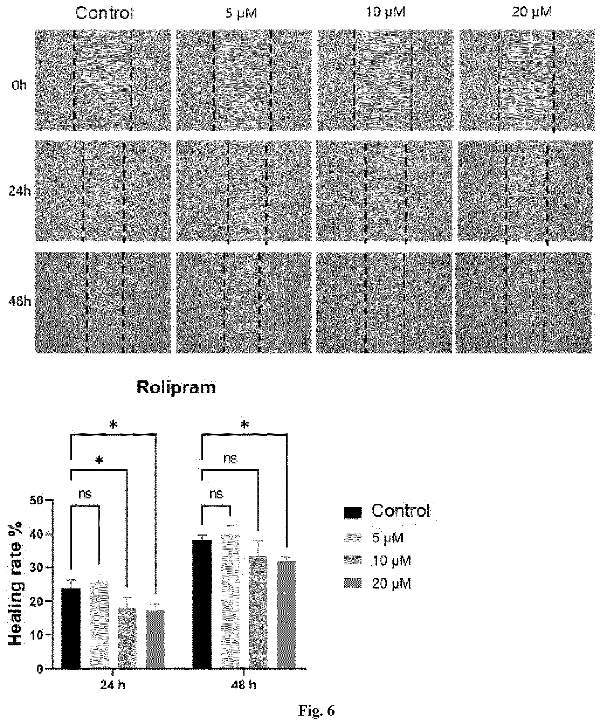

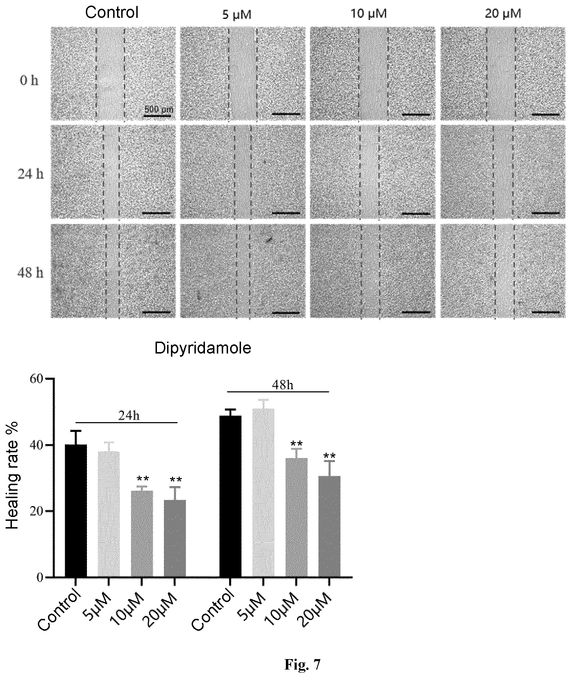

FIG. 3 shows the results of Roflumilast inhibiting metastasis. FIG. 4 shows the results of BPN14770 inhibiting metastasis. FIG. 5 shows GSK256066 inhibiting the results of metastasis. FIG. 6 shows the results of Rolipram inhibiting metastasis. FIG. 7 shows the results of Dipyridamole inhibiting metastasis. The results show that PDE4 inhibitors had a significant metastasis inhibition effect.

### Example 5 PDE4 Inhibitor Inhibiting Tumor Metastasis

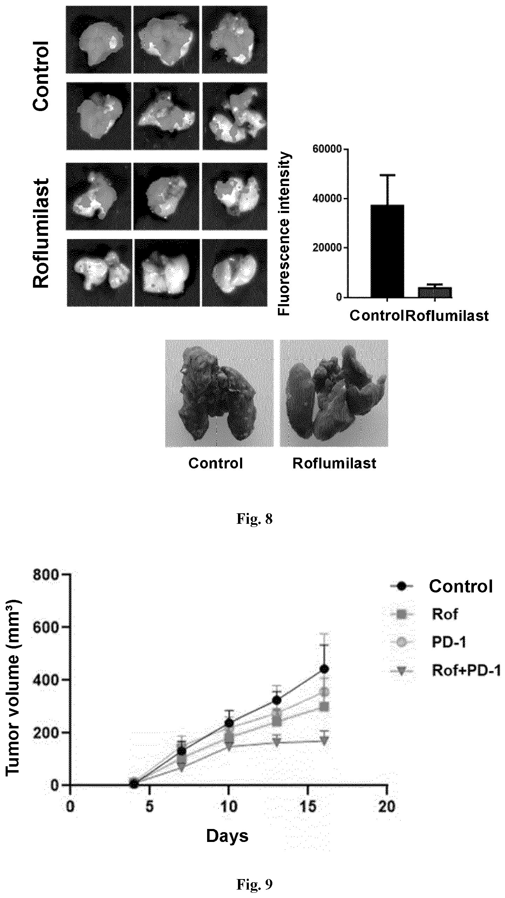

Roflumilast was administered in a mouse model of lung metastasis of breast cancer (using breast tumor cells 4T 1) to detect the inhibitory effect of a PDE4 inhibitor on tumor metastasis. FIG. 8 shows the inhibitory effect of Roflumilast on tumor metastasis. The results show that the PDE4 inhibitor had a significant inhibitory effect on tumor metastasis.

### Example 6 PDE4 Inhibitor In Combination With Immune Checkpoint For Inhibiting Tumors

In tumor models of mice, Roflumilast (Rof group), PD-1 antibody (InVivoMab, PD-1 group) or Roflumilast in combination with PD-1 antibody (Rof+PD-1 group) were administered to detect the inhibitory effect of PDE4 inhibitor in combination with immune checkpoint on tumors.

FIG. 9 shows the inhibitory effect of the PDE4 inhibitor in combination with an immune checkpoint on tumors. The results show that PDE4 inhibitors in combination with immune checkpoints had a significant synergistic anti-tumor effect.

### Example 7 PDE4 Knockout Inhibiting Metastasis

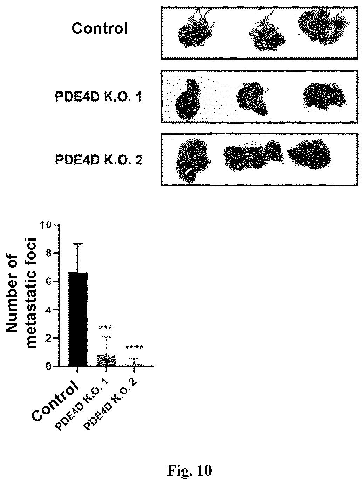

PDE4 is knocked out in a mouse model of colon cancer liver metastasis (using colon tumor cells MC38), e.g., by designing sgRNA according to the two regions of chr13: 108790711-110092503. As shown in FIG. 10, the PDE4 knockout can significantly inhibit the colon cancer liver metastasis.

The foregoing detailed description is provided by way of explanation and example and is not intended to limit the scope of the appended claims. Various variations of the implementations listed in the present application will be apparent to those of ordinary skill in the art and remain within the scope of the appended claims and their equivalents.

## Claims

1. A pharmaceutical combination comprising:
a) an effective amount of an immune checkpoint inhibitor; and
b) an effective amount of a PDE4-selective inhibitor.

2. The pharmaceutical combination of claim 1, wherein the PDE4-selective inhibitor comprises Apremilast, Crisaborole, Drotaverine, CC-1088, BPN14770, GSK356278, HT-0712, TAK-648, Zembrin, ASP9831, Etazolate, Piclamilast, Rolipram, Cilomilast, GSK256066, AWD 12-281, Quinolyl oxazole, DC-TA-46, Filaminast, and/or Glaucine.

3. The pharmaceutical combination of any one of claims 1-2, wherein the PDE4-selective inhibitor comprises a compound of Formula I, a pharmaceutically acceptable salt thereof, or an N-oxide of pyridine or a salt thereof: 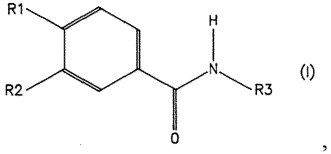 wherein one of R1 and R2 is hydrogen, C₁-C₆-alkoxy, C₃-C₇-cycloalkoxy, C₃-C₇-cycloalkylmethoxy, benzyloxy, or C₁-C₄-alkoxy fully or partially substituted with fluorine, and the other of R1 and R2 is C₁-C₄-alkoxy fully or partially substituted with fluorine, and
R3 is phenyl, pyridinyl, phenyl substituted with R31, R32 and R33, or pyridinyl substituted with R34, R35, R36 and R37, wherein R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

4. The pharmaceutical combination of claim 3, wherein:
R1 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

5. The pharmaceutical combination of claim 3, wherein:
R1 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine,
R2 is methoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

6. The pharmaceutical combination of claim 3, wherein:
R1 is C₃-C₇-cycloalkoxy, C₃-C₇-cycloalkylmethoxy or benzyloxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

7. The pharmaceutical combination of claim 3, wherein:
R1 is C₁-C₄-alkoxy fully or partially substituted with fluorine,
R2 is hydrogen, C₁-C₆-alkoxy, C₃-C₇-cycloalkoxy, C₃-C₇-cycloalkylmethoxy, benzyloxy or C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

8. The pharmaceutical combination of claim 3, wherein:
R1 is C₁-C₄-alkoxy fully or partially substituted with fluorine,
R2 is C₃-C₇-cycloalkylmethoxy or benzyloxy, and
R3 is phenyl, pyridinyl, phenyl substituted with R31, R32 and R33 or pyridinyl substituted with R34, R35, R36 and R37, wherein: R31 is hydroxyl, halogen, cyano, carboxyl, trifluoromethyl, C₁-C₄-alkyl, C₁-C₄-alkoxy, C₁-C₄-alkoxycarbonyl, C₁-C₄-alkylcarbonyl, C₁-C₄-alkylcarbonyloxy, amino, mono- or di-C₁-C₄-alkylamino or C₁-C₄-alkylcarbonylamino, R32 is hydrogen, hydroxyl, halogen, amino, trifluoromethyl, C₁-C₄-alkyl or C₁-C₄-alkoxy, R33 is hydrogen, halogen, C₁-C₄-alkyl or C₁-C₄-alkoxy, R34 is hydroxyl, halogen, cyano, carboxyl, C₁-C₄-alkyl, C₁-C₄-alkoxy, C₁-C₄-alkoxycarbonyl or amino, R35 is hydrogen, halogen, amino or C₁-C₄-alkyl, R36 is hydrogen or halogen, and R37 is hydrogen or halogen.

9. The pharmaceutical combination of claim 3, wherein:
R1 is C₁-C₄-alkoxy, Cs-Cs-cycloalkoxy, Cs-Cs-cycloalkylmethoxy, benzyloxy or C₁-C₄-alkoxy fully or partially substituted with fluorine,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

10. The pharmaceutical combination of claim 3, wherein:
R1 is C₁-C₄-alkoxy, Cs-Cs-cycloalkoxy, Cs-Cs-cycloalkylmethoxy, benzyloxy or C₁-C₄-alkoxy fully or partially substituted with fluorine,
R2 is methoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

11. The pharmaceutical combination of claim 3, wherein:
R1 is Cs-Cs-cycloalkoxy, Cs-Cs-cycloalkylmethoxy or benzyloxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

12. The pharmaceutical combination of claim 3, wherein:
R1 is C₁-C₄-alkoxy fully or partially substituted with fluorine,
R2 is C₁-C₄-alkoxy, Cs-Cs-cycloalkoxy, Cs-Cs-cycloalkylmethoxy, benzyloxy or C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

13. The pharmaceutical combination of claim 3, wherein:
R1 is C₁-C₄-alkoxy fully or partially substituted with fluorine,
R2 is Cs-Cs-cycloalkylmethoxy or benzyloxy, and
R3 is phenyl, pyridinyl, phenyl substituted with R31, R32 and R33 or pyridinyl substituted with R34, R35, R36 and R37, wherein R31 is halogen, cyano, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

14. The pharmaceutical combination of claim 3, wherein:
R1 is C₁-C₄-alkoxy, Cs-Cs-cycloalkoxy, Cs-Cs-cycloalkylmethoxy, benzyloxy or C₁-C₄-alkoxy fully or partially substituted with fluorine,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

15. The pharmaceutical combination of claim 3, wherein:
R1 is C₁-C₄-alkoxy, Cs-Cs-cycloalkoxy, Cs-Cs-cycloalkylmethoxy, benzyloxy or C₁-C₄-alkoxy fully or partially substituted with fluorine,
R2 is methoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

16. The pharmaceutical combination of claim 3, wherein:
R1 is Cs-Cs-cycloalkoxy, Cs-Cs-cycloalkylmethoxy or benzyloxy,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

17. The pharmaceutical combination of claim 3, wherein:
R1 is C₁-C₄-alkoxy fully or partially substituted with fluorine,
R2 is C₁-C₄-alkoxy, Cs-Cs-cycloalkoxy, Cs-Cs-cycloalkylmethoxy, benzyloxy or C₁-C₄-alkoxy fully or partially substituted with fluorine, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

18. The pharmaceutical combination of claim 3, wherein:
R1 is C₁-C₄-alkoxy fully or partially substituted with fluorine,
R2 is Cs-Cs-cycloalkylmethoxy or benzyloxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

19. The pharmaceutical combination of claim 3, wherein:
R1 is difluoromethoxy,
R2 is methoxy, ethoxy, isopropoxy, isobutoxy, cyclopentoxy, cyclopropylmethoxy, cyclobutylmethoxy, difluoromethoxy or 2,2,2-trifluoroethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

20. The pharmaceutical combination of claim 3, wherein:
R1 is difluoromethoxy,
R2 is methoxy, cyclopropylmethoxy, cyclobutylmethoxy or difluoromethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

21. The pharmaceutical combination of claim 3, wherein:
R1 is methoxy, n-propoxy, n-butoxy, cyclopropylmethoxy or 2,2,2-trifluoroethoxy,
R2 is difluoromethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

22. The pharmaceutical combination of claim 3, wherein:
R1 is difluoromethoxy,
R2 is cyclopropylmethoxy, and
R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3 -chloro-2,5,6-trifluoropyridin-4-yl, 3,5 -dichloro-2,6-difluoropyridin-4-yl, or 2,6-dichloropyridin-3-yl.

23. The pharmaceutical combination of claim 3, wherein:
R1 is C₃-C₇-cycloalkoxy or C₃-C₇-cycloalkylmethoxy, and R2 is C₁-C₄-alkoxy fully or partially substituted with fluorine; or R1 is C₁-C₄-alkoxy fully or partially substituted with fluorine, and R2 is C₃-C₇-cycloalkylmethoxy; and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C₄-alkyl, or C₁-C₄-alkoxy, R34 is hydroxy, halogen, carboxyl, C₁-C₄-alkyl, C₁-C₄-alkoxy, or C₁-C₄-alkoxycarbonyl, R35 is hydrogen, halogen, amino, or C₁-C₄-alkyl, R36 is hydrogen or halogen, and R37 is hydrogen or halogen.

24. The pharmaceutical combination of claim 3, wherein:
R1 is C₃-C₇-cycloalkoxy or C₃-C₇-cycloalkylmethoxy,
R2 is C₁-C₄-alkoxy fully or partially substituted with fluorine, and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C₄-alkyl, or C₁-C₄-alkoxy, R34 is hydroxy, halogen, carboxyl, C₁-C₄-alkyl, C₁-C₄-alkoxy, or C₁-C₄-alkoxycarbonyl, R35 is hydrogen, halogen, amino, or C₁-C₄-alkyl, R36 is hydrogen or halogen, and R37 is hydrogen or halogen.

25. The pharmaceutical combination of claim 3, wherein:
R1 is C₁-C₄-alkoxy fully or partially substituted with fluorine,
R2 is C₃-C₇-cycloalkylmethoxy, and
R3 is phenyl, pyridinyl, phenyl substituted with R31, R32, and R33, or pyridinyl substituted with R34, R35, R36, and R37, wherein: R31 is hydroxy, halogen, carboxyl, trifluoromethyl, C₁-C₄-alkyl, C₁-C₄-alkoxy, C₁-C₄-alkoxycarbonyl, or C₁-C₄-alkylcarbonyloxy, R32 is hydrogen, hydroxy, halogen, trifluoromethyl, C₁-C₄-alkyl, or C₁-C₄-alkoxy, R33 is hydrogen, halogen, C₁-C₄-alkyl, or C₁-C₄-alkoxy, R34 is hydroxy, halogen, carboxyl, C₁-C₄-alkyl, C₁-C₄-alkoxy, or C₁-C₄-alkoxycarbonyl, R35 is hydrogen, halogen, or C₁-C₄-alkyl, R36 is hydrogen or halogen, and R37 is hydrogen or halogen.

26. The pharmaceutical combination of claim 3, wherein:
R1 is C₃-C₅-cycloalkoxy or C₃-C₅-cycloalkylmethoxy,
R2 is C₁-C₄-alkoxy fully or partially substituted with fluorine, and
R3 is phenyl, pyridinyl, phenyl substituted with R31, R32 and R33 or pyridinyl substituted with R34, R35, R36 and R37, wherein R31 is halogen, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

27. The pharmaceutical combination of claim 3, wherein:
R1 is C₁-C₄-alkoxy fully or partially substituted with fluorine,
R2 is Cs-Cs-cycloalkylmethoxy, and
R3 is phenyl, pyridinyl, phenyl substituted with R31, R32 and R33 or pyridinyl substituted with R34, R35, R36 and R37, wherein R31 is halogen, carboxyl, C₁-C₄-alkyl, C₁-C₄-alkoxy or C₁-C₄-alkoxycarbonyl, R32 is hydrogen, halogen, C₁-C₄-alkyl or C₁-C₄-alkoxy, R33 is hydrogen, halogen, C₁-C₄-alkyl or C₁-C₄-alkoxy, R34 is halogen or C₁-C₄-alkyl, R35 is hydrogen or halogen, R36 is hydrogen or halogen, and R37 is hydrogen or halogen.

28. The pharmaceutical combination of claim 3, wherein:
R1 is Cs-Cs-cycloalkoxy, Cs-Cs-cycloalkylmethoxy or benzyloxy,
R2 is C₁-C₄-alkoxy fully or partially substituted with fluorine, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

29. The pharmaceutical combination of claim 3, wherein:
R1 is C₁-C₄-alkoxy fully or partially substituted with fluorine,
R2 is Cs-Cs-cycloalkylmethoxy, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

30. The pharmaceutical combination of claim 3, wherein:
R1 is difluoromethoxy,
R2 is cyclopropylmethoxy, and
R3 is 2-bromophenyl, 2,6-dichloro-4-ethoxycarbonylphenyl, 2,6-dimethoxyphenyl, 2,4,6-trifluorophenyl, 2-chloro-6-methylphenyl, 2,6-dimethylphenyl, 2,6-difluorophenyl, 2,6-dichlorophenyl, 3,5-dichloropyridin-4-yl, 3-methylpyridin-2-yl, 2-chloropyridin-3-yl, 3,5-dibromopyridin-2-yl, 2,3,5,6-tetrafluoropyridin-4-yl, 3-chloro-2,5,6-trifluoropyridin-4-yl, 3,5-dichloro-2,6-difluoropyridin-4-yl, or 2,6-dichloropyridin-3-yl.

31. The pharmaceutical combination of claim 3, wherein R1 is difluoromethoxy, R2 is cyclopropylmethoxy, and R3 is 3,5-dichloropyridin-4-yl.

32. The pharmaceutical combination of any one of claims 1-31, wherein the PDE4-selective inhibitor comprises Roflumilast or a derivative thereof.

33. The pharmaceutical combination of claim 32, wherein the derivative of Roflumilast comprises Benzanilide, 3-hydroxy-N-(3,5-dichloropyridin-4-yl)-4-methoxybenzamide and/or 4-Methylbenzanilide.

34. The pharmaceutical combination of any one of claims 1-33, wherein the immune checkpoint inhibitor comprises an antagonist of one or more proteins of CTLA-4, PD-1, PD-L1, PD-L2, LAG-3, TIM-3, galectin-9, CEACAM-1, BTLA, CD69, galectin-1, TIGIT, CD113, GPR56, VISTA, 2B4, CD48, GARP, PD1H, LAIR1, TIM-1, and TIM-4.

35. The pharmaceutical combination of any one of claims 1-34, wherein the immune checkpoint inhibitor comprises an antagonist of PD-1 and/or PD-L1.

36. The pharmaceutical combination of claim 35, wherein the antagonist of PD-1 and/or PD-L1 is capable of inhibiting an interaction between PD-1 and PD-L1.

37. The pharmaceutical combination of any one of claims 35-36, wherein the antagonist of PD-1 and/or PD-L1 comprises an antibody specifically binding to PD-1 and/or PD-L1 or an antigen binding fragment thereof.

38. The pharmaceutical combination of claim 37, wherein the antibody specifically binding to PD-1 and/or PD-L1 or antigen binding fragment thereof comprises a HCDR3 comprising an amino acid sequence set forth in any one of SEQ ID NOs: 3, 13, 23, and 33.

39. The pharmaceutical combination of any one of claims 37-38, wherein the antibody specifically binding to PD-1 and/or PD-L1 or antigen binding fragment thereof comprises a HCDR2 comprising an amino acid sequence set forth in any one of SEQ ID NOs: 2, 12, 22, and 32.

40. The pharmaceutical combination of any one of claims 37-39, wherein the antibody specifically binding to PD-1 and/or PD-L1 or antigen binding fragment thereof comprises a HCDR1 comprising an amino acid sequence set forth in any one of SEQ ID NOs: 1, 11, 21, and 31.

41. The pharmaceutical combination of any one of claims 37-40, wherein the antibody specifically binding to PD-1 and/or PD-L1 or antigen binding fragment thereof comprises a VH comprising an amino acid sequence set forth in any one of SEQ ID NOs: 7, 17, 27, and 37.

42. The pharmaceutical combination of any one of claims 37-41, wherein the antibody specifically binding to PD-1 and/or PD-L1 or antigen binding fragment thereof comprises an LCDR3 comprising an amino acid sequence set forth in any one of SEQ ID NOs: 6, 16, 26, and 36.

43. The pharmaceutical combination of any one of claims 37-42, wherein the antibody specifically binding to PD-1 and/or PD-L1 or antigen binding fragment thereof comprises an LCDR2 comprising an amino acid sequence set forth in any one of SEQ ID NOs: 5, 15, 25, and 35.

44. The pharmaceutical combination of any one of claims 37-43, wherein the antibody specifically binding to PD-1 and/or PD-L1 or antigen binding fragment thereof comprises an LCDR1 comprising an amino acid sequence set forth in any one of SEQ ID NOs: 4, 14, 24, and 34.

45. The pharmaceutical combination of any one of claims 37-44, wherein the antibody specifically binding to PD-1 and/or PD-L1 or antigen binding fragment thereof comprises a VL comprising an amino acid sequence set forth in any one of SEQ ID NOs: 8, 18, 28, and 38.

46. The pharmaceutical combination of any one of claims 37-45, wherein the antibody specifically binding to PD-1 and/or PD-L1 is selected from the group consisting of:
a) an antibody comprising the following heavy chain and light chain, the heavy chain comprises an amino acid sequence set forth in any one of SEQ ID NOs: 9, 19, 29, and 39, and the light chain comprises an amino acid sequence set forth in any one of SEQ ID NOs: 10, 20, 30, and 40;
b) an antibody comprising the following heavy chain and light chain, the heavy chain comprises a VH having an amino acid sequence set forth in any one of SEQ ID NOs: 7, 17, 27 and 37, and the light chain comprises a VL having an amino acid sequence set forth in any one of SEQ ID NOs: 8, 18, 28 and 38; and
c) an antibody comprising the following heavy chain and light chain, the heavy chain comprises a HCDR1 having an amino acid sequence set forth in any one of SEQ ID NOs: 1, 11, 21, and 31, a HCDR2 having an amino acid sequence set forth in any one of SEQ ID NOs: 2, 12, 22, and 32, and a HCDR3 having an amino acid sequence set forth in any one of SEQ ID NOs: 3, 13, 23, and 33, and the light chain comprises an LCDR1 having an amino acid sequence set forth in any one of SEQ ID NOs: 4, 14, 24, and 34, an LCDR2 having an amino acid sequence set forth in any one of SEQ ID NOs: 5, 15, 25, and 35, and an LCDR3 having an amino acid sequence set forth in any one of SEQ ID NOs: 6, 16, 26, and 36.

47. The pharmaceutical combination of any one of claims 37-46, wherein the antibody is selected from: a chimeric antibody, a humanized antibody, and a fully human antibody.

48. The pharmaceutical combination of any one of claims 37-47, wherein the antigen binding fragment is selected from: Fab, Fv, scFv, Fab 'and (Fab')₂.

49. The pharmaceutical combination of any one of claims 37-48, wherein the antibody specifically binding to PD-1 and/or PD-L1 comprises:
HCDR1-3 and LCDR1-3 of nivolumab,
HCDR1-3 and LCDR1-3 of pembrolizumab,
HCDR1-3 and LCDR1-3 of BMS-936559;
HCDR1-3 and LCDR1-3 of MPDL3280A;
HCDR1-3 and LCDR1-3 of MEDI4736; or
HCDR1-3 and LCDR1-3 of MSB0010718C.

50. The pharmaceutical combination of any one of claims 37-49, wherein the antibody specifically binding to PD-1 and/or PD-L1 comprises:
a VH and a VL of nivolumab,
a VH and a VL of pembrolizumab,
a VH and a VL of BMS-936559;
a VH and a VL of MPDL3280A;
a VH and a VL of MEDI4736; or
a VH and a VL of MSB0010718C.

51. The pharmaceutical combination of any one of claims 37-50, wherein the antibody specifically binding to PD-1 and/or PD-L1 is selected from the group consisting of nivolumab, pembrolizumab, BMS-936559, MPDL3280A, MEDI4736, AMP-224, and MSB0010718C.

52. The pharmaceutical combination of any one of claims 1-51, wherein a ratio of the effective amount of the PDE4-selective inhibitor to the effective amount of the immune checkpoint inhibitor is from about 9:1 to about 1:4 (mass ratio).

53. The pharmaceutical combination of any one of claims 1-52, wherein the PDE4-selective inhibitor and the immune checkpoint inhibitor are present in different containers, respectively.

54. The pharmaceutical combination of any one of claims 1-53, wherein the pharmaceutical combination comprises a first formulation and a second formulation, the first formulation comprises the immune checkpoint inhibitor and a first pharmaceutically acceptable carrier, and the second formulation comprises the PDE4-selective inhibitor and a second pharmaceutically acceptable carrier.

55. The pharmaceutical combination of claim 54, wherein the PDE4-selective inhibitor is contained in the second formulation in an amount of about 20% (w/w) to about 90% (w/w).

56. The pharmaceutical combination of any one of claims 54-55, wherein the immune checkpoint inhibitor is contained in the first formulation in an amount of about 10% (w/w) to about 80% (w/w).

57. The pharmaceutical combination of any one of claims 1-56, comprising a pharmaceutical composition, and the pharmaceutical composition comprises the PDE4-selective inhibitor and the immune checkpoint inhibitor.

58. The pharmaceutical combination of claim 57, wherein the PDE4-selective inhibitor is contained in the pharmaceutical composition in an amount of about 20% (w/w) to about 90% (w/w).

59. The pharmaceutical combination of any one of claims 57-58, wherein the immune checkpoint inhibitor is contained in the pharmaceutical composition in an amount of about 10% (w/w) to about 80% (w/w).

60. A kit comprising the pharmaceutical combination of any one of claims 1-59.

61. The pharmaceutical combination of any one of claims 1-59 or the kit of claim 60 for preventing and/or treating a tumor.

62. The pharmaceutical combination or kit of claim 61, wherein the tumor comprises a solid tumor, a hematological tumor, and/or a lymphoma.

63. The pharmaceutical combination or kit of any one of claims 61-62, wherein the tumor comprises colon cancer, breast cancer, and/or melanoma.

64. The pharmaceutical combination or kit of any one of claims 61-63, wherein the tumor comprises a metastatic tumor.

65. The pharmaceutical combination or kit of any one of claims 61-64, wherein the tumor comprises a tumor with liver metastasis.

66. The pharmaceutical combination or kit of any one of claims 61-65, wherein the tumor comprises colon cancer with liver metastasis.

67. A PDE4-selective inhibitor for preventing and/or treating a tumor.

68. The PDE4-selective inhibitor of claim 67 for preventing and/or treating a tumor in combination with the immune checkpoint inhibitor of any one of claims 1-66.

69. The PDE4-selective inhibitor of any one of claims 67-68, wherein the PDE4-selective inhibitor is the PDE4-selective inhibitor of any one of claims 1-66.

70. The PDE4-selective inhibitor of any one of claims 67-69, wherein the tumor comprises a solid tumor, a hematological tumor, and/or a lymphoma.

71. The PDE4-selective inhibitor of any one of claims 67-70, wherein the tumor comprises colon cancer, breast cancer, and/or melanoma.

72. The PDE4-selective inhibitor of any one of claims 67-71, wherein the tumor comprises a metastatic tumor.

73. The PDE4-selective inhibitor of any one of claims 67-72, wherein the tumor comprises a tumor with liver metastasis.

74. The PDE4-selective inhibitor of any one of claims 67-73, wherein the tumor comprises colon cancer with liver metastasis.

75. A method for preventing and/or treating a tumor, comprising administering to a subject in need thereof an effective amount of the PDE4-selective inhibitor of any one of claims 1-74.

76. The method of claim 75, further comprising administering to the subject an effective amount of the immune checkpoint inhibitor of any one of claims 1-66.

77. The method of claim 76, wherein the PDE4-selective inhibitor is administered simultaneously or sequentially with the immune checkpoint inhibitor.

78. The method of any one of claims 75-77, wherein the tumor comprises a solid tumor, a hematological tumor, and/or a lymphoma.

79. The method of any one of claims 75-78, wherein the tumor comprises colon cancer, breast cancer, and/or melanoma.

80. The method of any one of claims 75-79, wherein the tumor comprises a metastatic tumor.

81. The method of any one of claims 75-80, wherein the tumor comprises a tumor with liver metastasis.

82. The method of any one of claims 75-81, wherein the tumor comprises colon cancer with liver metastasis.

83. Use of a PDE4-selective inhibitor in preparation of a medicament for preventing and/or treating a tumor.

84. Use of a PDE4-selective inhibitor in combination with an immune checkpoint inhibitor in preparation of a medicament for preventing and/or treating a tumor.

85. The use of any one of claims 83-84, wherein the PDE4-selective inhibitor is the PDE4-selective inhibitor of any one of claims 1-74.

86. The use of any one of claims 83-85, wherein the immune checkpoint inhibitor is the immune checkpoint inhibitor of any one of claims 1-66.

87. The use of any one of claims 83-86, wherein the tumor comprises a solid tumor, a hematological tumor, and/or a lymphoma.

88. The use of any one of claims 83-87, wherein the tumor comprises colon cancer, breast cancer, and/or melanoma.

89. The use of any one of claims 83-88, wherein the tumor comprises a metastatic tumor.

90. The use of any one of claims 83-89, wherein the tumor comprises a tumor with liver metastasis.

91. The use of any one of claims 83-90, wherein the tumor comprises colon cancer with liver metastasis.
